# EUROPEAN PATENT SPECIFICATION

(11) **EP 2 882 707 B1**
(45) Date of publication and mention of the grant of the patent: **08.03.2017**
(21) Application number: 13827088.9
(22) Date of filing: 18.07.2013
(51) Int. Cl.: C07C 255/51, C07D 235/18, C09K 11/06, H01L 51/50, C07C 255/52, C07D 215/12, C07D 217/18, C07D 235/14, C07D 209/86, C07C 13/62, C07D 213/24, C07D 213/53, C07D 213/57, G03G 15/04, H01L 51/00, H05B 33/08, H05B 33/14

(54) **NOVEL FUSED POLYCYCLIC COMPOUND AND ORGANIC LIGHT-EMITTING DEVICE, DISPLAY APPARATUS, IMAGE INFORMATION PROCESSING APPARATUS, LIGHTING SYSTEM, AND IMAGE FORMING APPARATUS HAVING THE COMPOUND**
NEUE KONDENSIERTE POLYZYKLISCHE VERBINDUNG UND ORGANISCHE LICHTEMITTIERENDE VORRICHTUNG, ANZEIGEVORRICHTUNG, BILDINFORMATIONSVERARBEITUNGSVORRICHTUNG, BELEUCHTUNGSSYSTEM SOWIE BILDERZEUGUNGSVORRICHTUNG MIT DIESER VERBINDUNG
NOUVEAU COMPOSÉ POLYCYCLIQUE CONDENSÉ ET DISPOSITIF ÉLECTROLUMINESCENT ORGANIQUE, APPAREIL D'AFFICHAGE, APPAREIL DE TRAITEMENT D'INFORMATIONS D'IMAGE, SYSTÈME D'ÉCLAIRAGE, ET APPAREIL DE FORMATION D'IMAGE AYANT LE COMPOSÉ

(30) Priority: 07.08.2012 JP 2012174880
(43) Date of publication of application: 17.06.2015
(73) Proprietor: Canon Kabushiki Kaisha, Tokyo 146-8501 (JP)
(72) Inventor: TAGAMI, Kei, Tokyo 146-8501 (JP); OHRUI, Hiroki, Tokyo 146-8501 (JP); IWAWAKI, Hironobu, Tokyo 146-8501 (JP); ITABASHI, Masumi, Tokyo 146-8501 (JP); TAKAHASHI, Tetsuo, Tokyo 146-8501 (JP); IKARI, Kenichi, Tokyo 146-8501 (JP); ISHII, Ryuji, Tokyo 146-8501 (JP); MURATSUBAKI, Masanori, Tokyo 146-8501 (JP)
(74) Representative: TBK
(86) International application number: PCT/JP2013/070127
(87) International publication number: WO 2014/024687

(56) References cited:
- WO-A1-2010/067757
- WO-A1-2010/071224
- WO-A1-2010/123153
- JP-A- H10 294 177
- JP-A- 2005 053 806

## Description

### Technical Field

The present invention relates to a novel fused polycyclic compound and an organic light-emitting device, a display apparatus, an image information processing apparatus, a lighting system, and an image forming apparatus each including the compound.

### Background Art

Organic light-emitting devices each have a pair of electrodes and an organic compound layer containing a fluorescent or phosphorescent organic compound disposed between the electrodes, generate excitons of the fluorescent or phosphorescent organic compound by injection of electrons and holes from the electrodes, and utilize the light emitted when the excitons return to the ground state.

In recent organic light-emitting devices, low driving voltages, high luminance, various emission wavelengths, rapid response, and reductions in size and weight are possible, and wide application of the light-emitting devices is suggested.

PTL 1 describes naphtho[2,3-k]fluoranthene as a compound for organic EL devices. PTL 2 describes naphthofluoranthene as a light-emitting material for organic electroluminescent devices.

Naphtho[2,3-k]fluoranthene described in PTL 1 has a peak emission wavelength of 470 nm, which is the longer wavelength side as a blue light, and is therefore an undesirable compound as a blue light-emitting material.

Naphthofluoranthene described in PTL 2 has a small difference between the first and second peak emission wavelengths, resulting in a low color purity of light emission.

The organic compounds and the organic light-emitting devices using the compounds described in these patent documents need to be further improved, from the viewpoint of practical application.

Specifically, application to practical use requires a further increase in luminance of optical output or in conversion efficiency. Furthermore, in application to, for example, a full color display, the organic light-emitting device is required to emit blue light with a high color purity and a high efficiency. These issues have not sufficiently been resolved yet.

Though PTL 1 and PTL 2 as well as PTL 3 describe light-emitting materials used in organic light-emitting devices, there is a demand for a light-emitting material having a further high color purity and a further high efficiency.

### Citation List

### Patent Literature

PTL 1 Japanese Patent Laid-Open No. 2005-53806
PTL 2 Japanese Patent Laid-Open No. 10-294177
PTL 3 WO 2010/067757

### Summary of Invention

The present invention provides a novel fused polycyclic compound that emits blue light with a high color purity and a high luminous efficiency. The present invention provides an organic light-emitting device, a display apparatus, an image information processing apparatus, a lighting system, and an image forming apparatus each including the compound.

The present invention provides a fused polycyclic compound represented by the following Formula [1]: 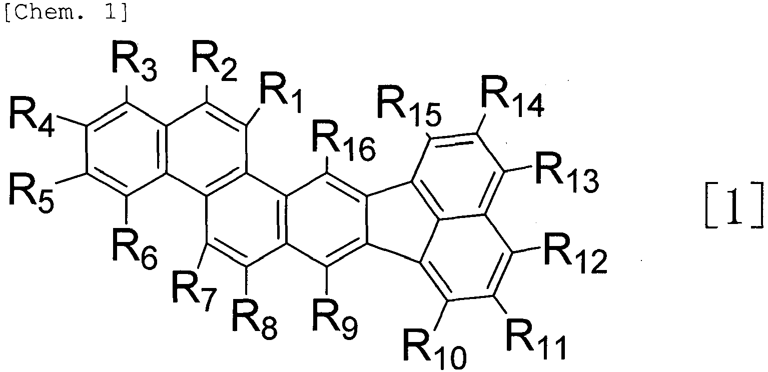 wherein R₁ to R₁₆ each independently represent a group selected from a hydrogen atom, a cyano group, substituted or unsubstituted alkyl groups, substituted or unsubstituted aryl groups, and substituted or unsubstituted heterocyclic groups.

Further features of the present invention will become apparent from the following description of exemplary embodiments with reference to the attached drawings.

### Advantageous Effects of Invention

The present invention can provide a novel fused polycyclic compound that emits blue light having a high color purity with a high luminous efficiency and also can provide an organic light-emitting device including the compound so as to have a high luminous efficiency and a long life.

### Brief Description of Drawings

Fig. 1 is a schematic cross-sectional view illustrating an example of a display apparatus including an organic light-emitting device of the present invention and an active device connected to the organic light-emitting device.
Fig. 2 is a graph showing photoluminescence spectra of Example Compound D16 and Comparative Compound R₁.

### Description of Embodiment

The present invention relates to a fused polycyclic compound represented by the following Formula [1]: 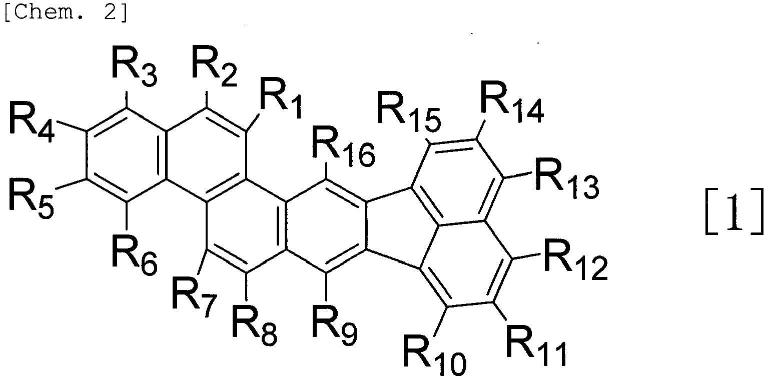 wherein R₁ to R₁₆ each independently represent a group selected from a hydrogen atom, a cyano group, substituted or unsubstituted alkyl groups, substituted or unsubstituted aryl groups, and substituted or unsubstituted heterocyclic groups.

The fused polycyclic compound of the present invention is also called a acenaphtho[1,2-b]chrysene compound. Hereinafter, the fused polycyclic compound of the present invention is also called an acenaphtho[1,2-b]chrysene compound in the embodiment.

In Formula [1], at least two of R₂, R₄, R₈, R₁₂, and R₁₃ can be electron-withdrawing substituents.

In Formula [1], examples of the substituted or unsubstituted alkyl group include, but not limited to, methyl, ethyl, n-propyl, n-butyl, n-pentyl, n-hexyl, n-heptyl, n-octyl, n-decyl, iso-propyl, iso-butyl, sec-butyl, tert-butyl, iso-pentyl, neopentyl, tert-octyl, fluoromethyl, difluoromethyl, trifluoromethyl, 2-fluoroethyl, 2,2,2-trifluoroethyl, perfluoroethyl, 3-fluoropropyl, perfluoropropyl, 4-fluorobutyl, perfluorobutyl, 5-fluoropentyl, 6-fluorohexyl, cyclopropyl, cyclobutyl, cyclopentyl, cyclohexyl, cyclopentylmethyl, cyclohexylmethyl, cyclohexylethyl, 4-fluorocyclohexyl, norbornyl, and adamantly groups.

In Formula [1], examples of the aryl group include, but not limited to, phenyl, naphthyl, indenyl, biphenyl, terphenyl, fluorenyl, anthryl, phenanthryl, pyrenyl, tetracenyl, pentacenyl, triphenyl, and perylenyl groups.

In Formula [1], examples of the heterocyclic group include, but not limited to, thienyl, pyrrolyl, pyridyl, pyrazyl, pyrimidyl, pyridazinyl, quinolinyl, isoquinolinyl, oxazolyl, oxadiazolyl, phenanthridinyl, acridinyl, naphthyridinyl, quinoxalinyl, quinazolinyl, cinnolinyl, phthalazinyl, phenanthrolyl, phenazinyl, dibenzofuranyl, dibenzothiophenyl, carbazolyl, benzofuranyl, benzothiophenyl, indolyl, cycloazyl, benzoimidazolyl, benzothiazolyl, and benzothiadiazolyl groups.

In Formula [1], examples of the substituent, i.e., the substituent possessed by the alkyl group, the aryl group, or the heterocyclic group, include, but not limited to, alkyl groups such as methyl, ethyl, propyl, and butyl groups; aralkyl groups such as a benzyl group; aryl groups such as phenyl and biphenyl groups; heterocyclic groups such as pyridyl, pyrrolyl, benzoimidazolyl, and benzothiazolyl groups; amino groups such as dimethylamino, diethylamino, dibenzylamino, diphenylamino, and ditolylamino groups; alkoxyl groups such as methoxyl, ethoxyl, propoxyl, and phenoxyl groups; a cyano group; and halogen atoms such as fluorine, chlorine, bromine, and iodine.

In Formula [1], examples of the electron-withdrawing substituent include, but not limited to, cyano, nitro, alkylsulfonyl, halogenated alkyl, acyl, alkoxycarbonyl, sulfamoyl, carbamoyl, halogenated alkoxy, sulfonyloxy, halogenated alkylthio, benzoxazolyl, benzothiazolyl, benzimidazolyl, and oxazolopyridyl groups and halogen atoms.

In this embodiment, a basic skeleton refers to a structure formed of only fused rings. In the acenaphtho[1,2-b]chrysene compound of the present invention, the structure of the compound represented by Formula [1] and having all of R₁ to R₁₆ being hydrogen atoms corresponds to the basic skeleton of the present invention.

The luminous efficiency of an organic light-emitting device can be enhanced by increasing the light emission quantum efficiency of an organic compound of an emission center.

An organic compound having a high quantum efficiency is required to satisfy the following two requirements:
1. the oscillator strength is high, and
2. the number of oscillating portion of a skeleton involved in light emission is small.
Satisfaction of the requirement 1, i.e., a high oscillator strength leads to a high quantum efficiency.

In a skeleton having a high oscillator strength, the symmetry of the skeleton involved in light emission of a molecule is high. However, such a highly symmetric molecule may not emit light under the forbidden transition condition peculiar to the molecule.

The oscillator strength can also be improved by increasing the dipole moment of a molecule, the dipole moment being increased by further extending the conjugate using the longer direction of the conjugate plane as an axis.

The acenaphtho[1,2-b]chrysene compound of the present invention has a structure having a fused-ring in the direction extending the conjugate from the 10-position to the 11-position of naphtho[1,2-k]fluoranthene.

This structure allows the [1,2-b]chrysene compound of the present invention to have a further larger dipole moment than that of naphtho[1,2-k]fluoranthene and is a structure having a higher oscillator strength than that of naphtho[1,2-k]fluoranthene. 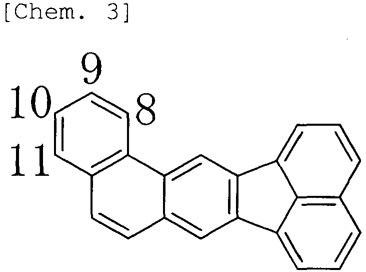 Naphtho[1,2-k]fluoranthene

The oscillator strengths of structures having benzene rings fused to the 8- and 9-positions, 9- and 10-positions, or 10- and 11-positions of a naphtho[1,2-k]fluoranthene skeleton, a naphtho[2,3-k]fluoranthene skeleton, and a naphtho[1,2-k]fluoranthene skeleton were calculated.

Based on the density functional theory, quantum chemical calculation at a B3LYP/6-31G* level was used.

The results are shown in Table 1.

**[Table 1]**

| | Structure | Oscillator strength |
|---|---|---|
| Naphtho[1,2-k]fluoranthene | 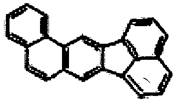 | 0.140 |
| Naphtho[2,3-k]fluoranthene | 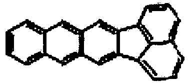 | 0.236 |
| Phenanthro[3,4-k]fluoranthene | 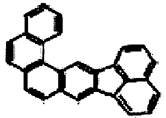 | 0.199 |
| Acenaphtho[1,2-b]tetraphene | 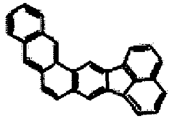 | 0.046 |
| Acenaphtho[1,2-b]chrysene | 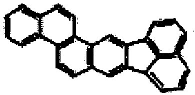 | 0.252 |

The oscillator strength of the acenaphtho[1,2-b]chrysene skeleton of the present invention is the highest among those of the three structures each having a benzene ring fused to the naphtho[1,2-k]fluoranthene skeleton and is higher than that of naphtho[2,3-k]fluoranthene.

Satisfaction of the requirement 2 means that the skeleton involved in light emission does not have any rotational structure, leading to suppression of a decrease in quantum efficiency due to rotational oscillation.

The basic skeleton of the acenaphtho[1,2-b]chrysene compound of the present invention does not have any rotational structure, and the quantum efficiency is therefore prevented from being decreased due to rotational oscillation.

Incidentally, physical properties required in materials suitable for blue light emission as an organic EL display are emission peaks. That is, the blue-light-emitting material is required to have an emission peak of 460 nm or less, in particular, an emission peak of 450 nm or less as a blue-light-emitting material with a high color purity.

A blue-light-emitting material having an emission peak of 450 nm or less can be provided by appropriately selecting the position of a substituent and the type of the substituent introduced into the acenaphtho[1,2-b]chrysene skeleton of the basic skeleton of the fused polycyclic compound according to the present invention.

The basic skeleton of the fused polycyclic compound of the present invention has high flatness and easily forms excimers due to intermolecular stacking when it is unsubstituted.

Accordingly, in order to inhibit the formation of excimers, the fused polycyclic compound of the present invention has a substituent, such as an aryl group, a heterocyclic group, or an alkyl group, at R₂, R₄, R₈, R₁₂, or R₁₃ in Formula [1].

The present invention was made through molecular design based on the consideration above.

The fused polycyclic compound of the present invention has a high color purity and a high quantum efficiency and can therefore be used as a light-emitting material for organic light-emitting devices.

In particular, the compound can be used as a material that emits blue light.

In the embodiment, light in a blue region refers to emission of light with a wavelength of 425 nm or more and 460 nm or less.

Examples of structural formula of the acenaphtho[1,2-b]chrysene compound of the present invention are specifically shown below:

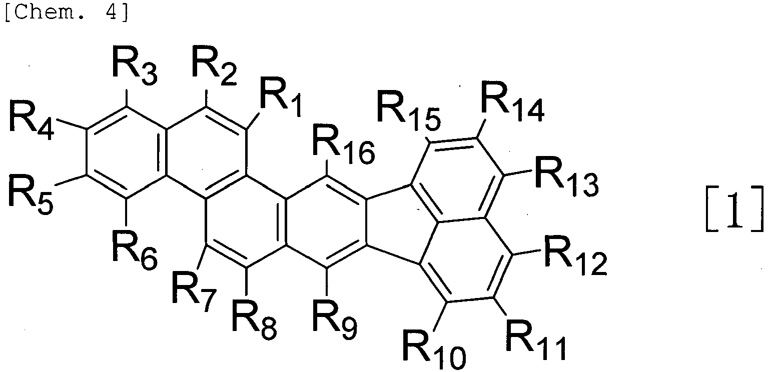

The following Formulae D1 to D3 show examples of the compound represented by Formula [1], wherein R₁ to R₁₆ are each selected from a hydrogen atom and substituted or unsubstituted alkyl groups.

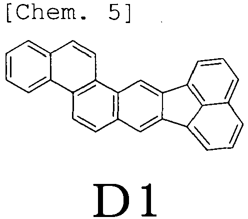

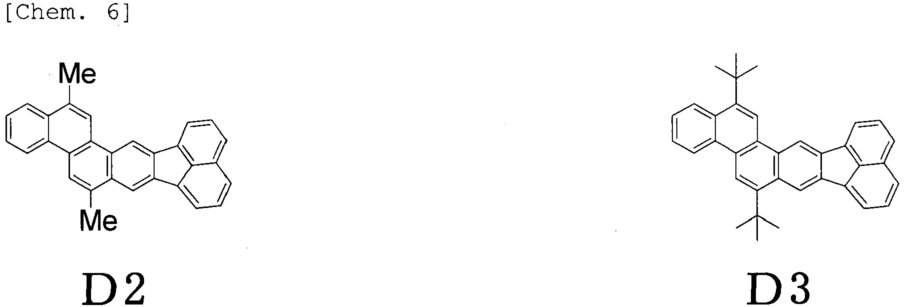

The following Formulae D4 to D11 show examples of the compound represented by Formula [1], wherein two of R₁ to R₁₆ are each an unsubstituted aryl group.

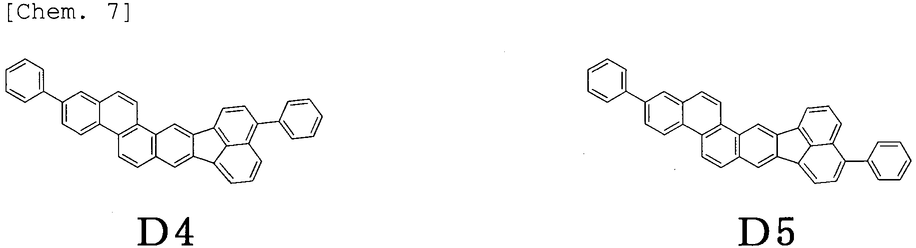

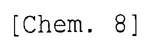

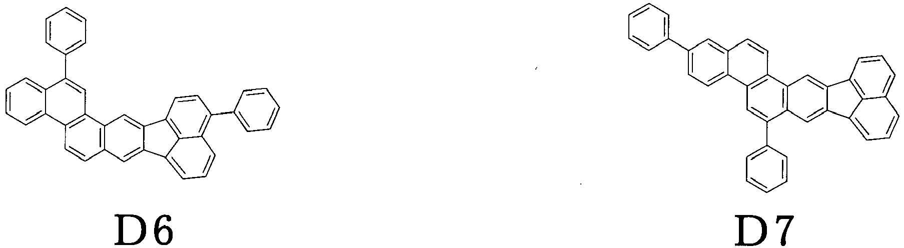

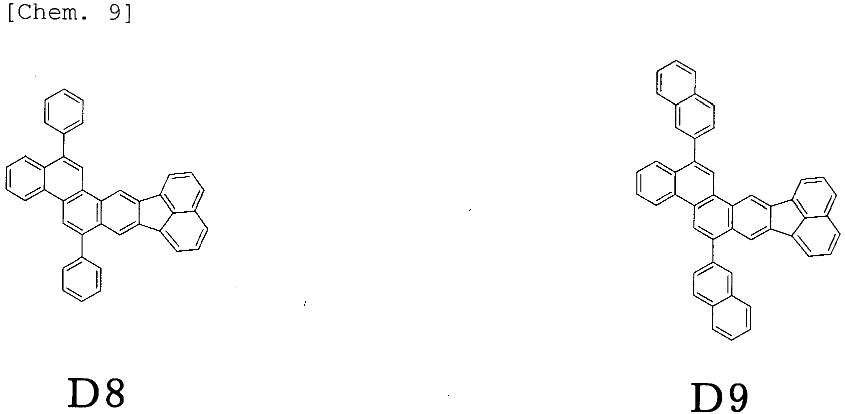

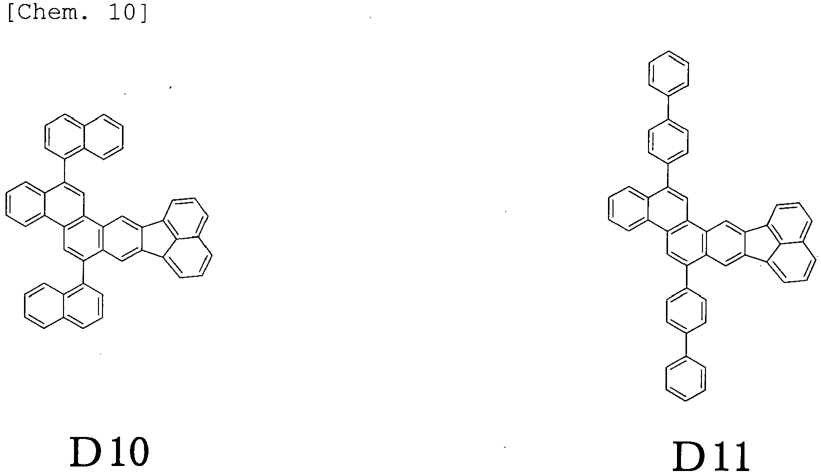

The acenaphtho[1,2-b]chrysene compound according to the present invention can be a fused polycyclic compound represented by the following Formula [2]: 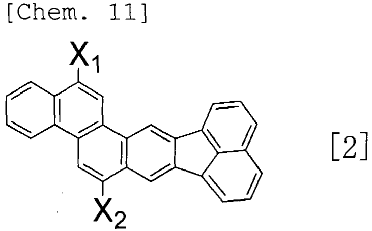 wherein X₁ and X₂ each independently represent a group selected from a substituted aryl groups, and substituted or unsubstituted heterocyclic groups.

The substituents optionally possessed by the aryl groups, and the heterocyclic groups are the same as those optionally possessed by the substituents in Formula [1].

The following Formulae D12 to D15 show examples of the compound represented by Formula [2], wherein X₁ and X₂ are each an aryl group substituted with an alkyl group such as a methyl or butyl group.

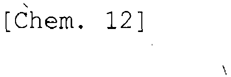

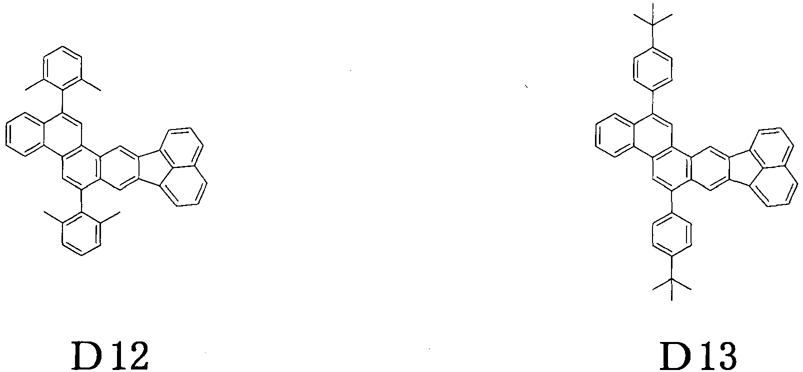

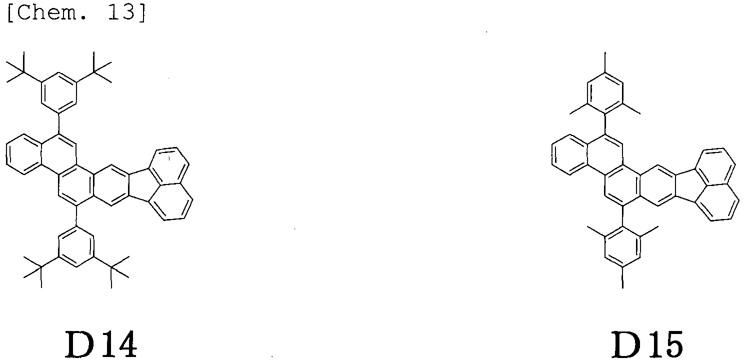

The following Formulae D16 to D21 show examples of the compound represented by Formula [2], wherein X₁ and X₂ are each an aryl group substituted with a cyano group or an alkyl group such as a methyl group.

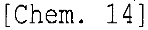

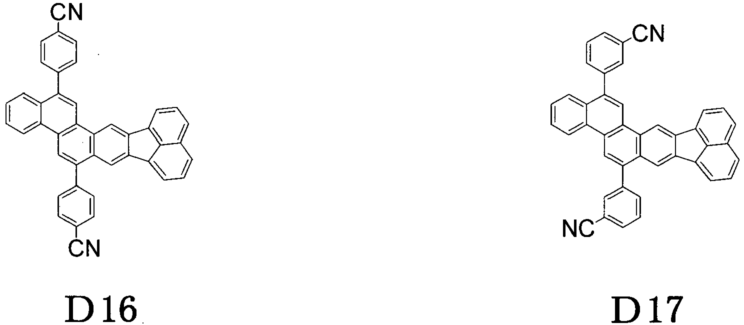

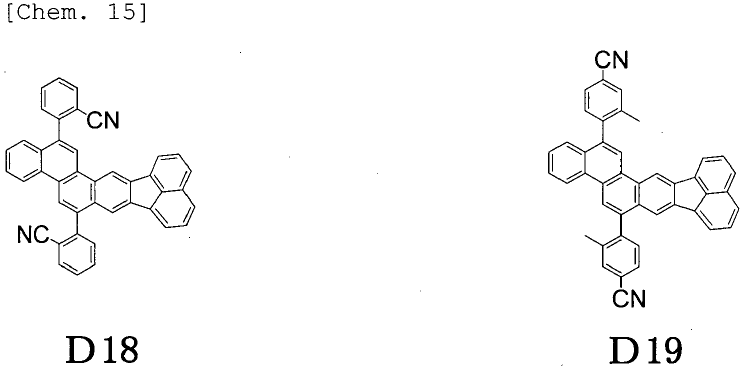

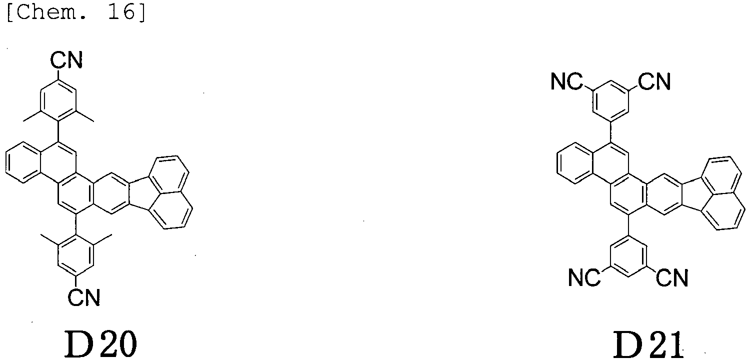

The following Formulae D22 to D29 show examples of the compound represented by Formula [2], wherein X₁ and X₂ are each a substituted or unsubstituted heterocyclic group.

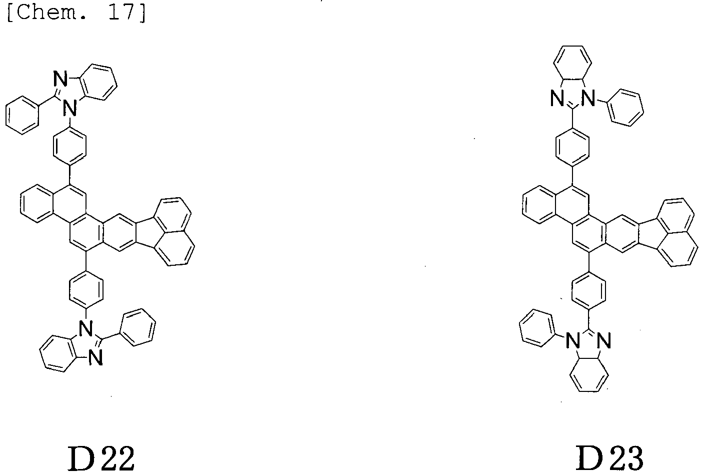

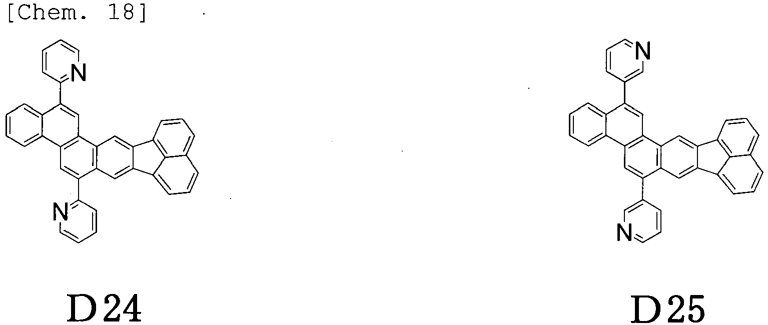

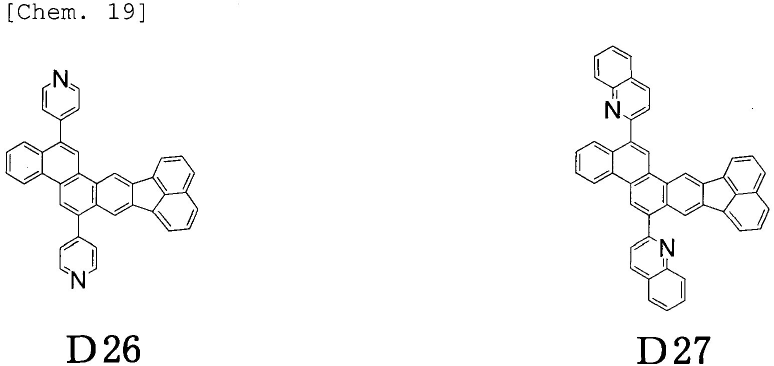

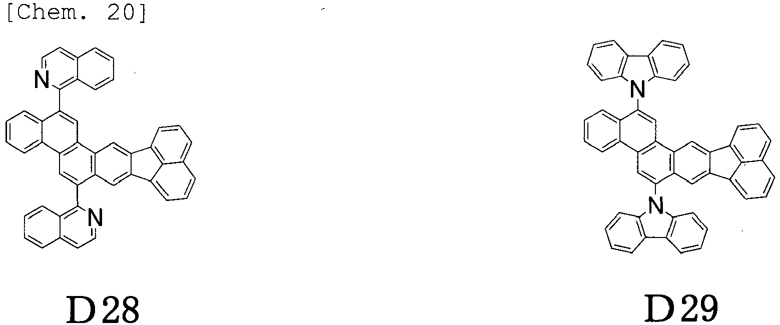

The fused polycyclic compound of the present invention can be a compound represented by the following Formula [3] or [4]: 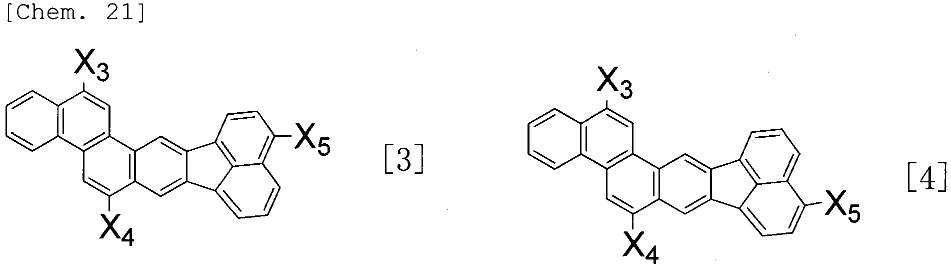 wherein X₃ to X₅ each independently represent a group selected from substituted or unsubstituted aryl groups, and substituted or unsubstituted heterocyclic groups.

The substituents optionally possessed by the aryl groups, and the heterocyclic groups are the same as those optionally possessed by the substituents in Formula [1].

The following Formulae D30 and D31 show examples of the compound represented by Formula [3] or [4], wherein X₃ to X₅ are each an unsubstituted aryl group.

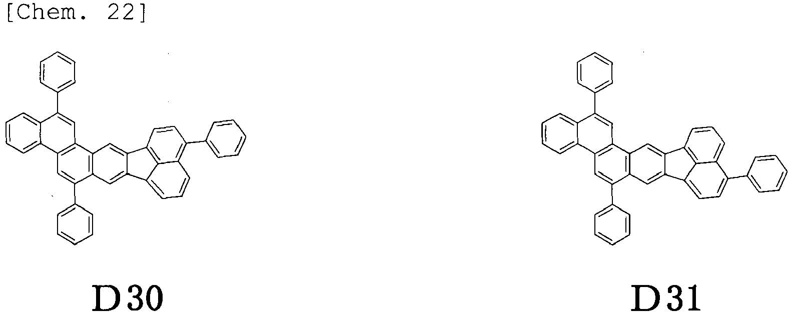

The following Formulae D32 and D33 show examples of the compound represented by Formula [3] or [4], wherein X₃ and X₄ are each an unsubstituted aryl group; and X₅ is a substituted or unsubstituted heterocyclic group.

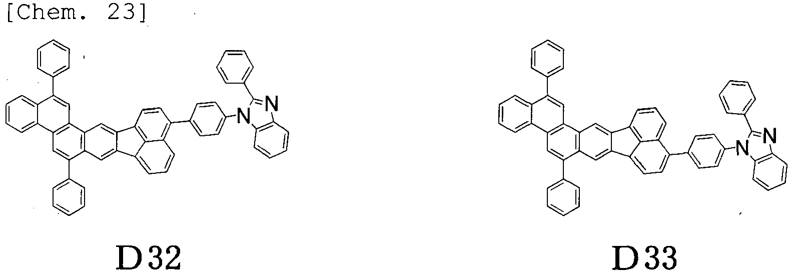

The following Formulae D34 to D51 show examples of the compound represented by Formula [3] or [4], wherein X₃ and X₄ are each an aryl group substituted with a cyano group or a methyl group; and X₅ is an aryl group substituted with a methyl group or an unsubstituted aryl group.

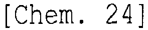

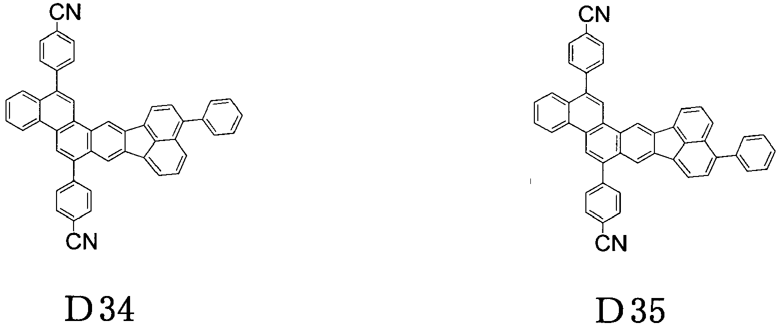

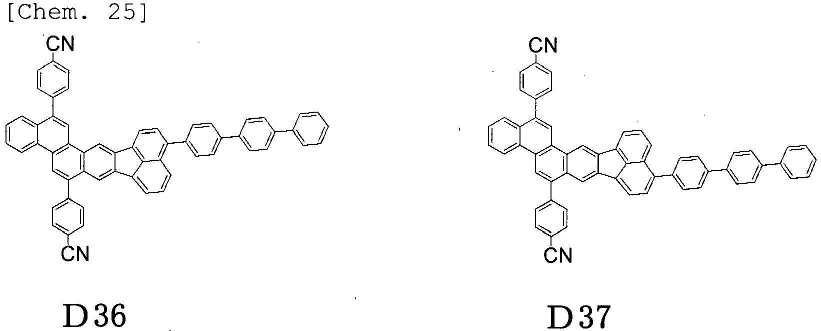

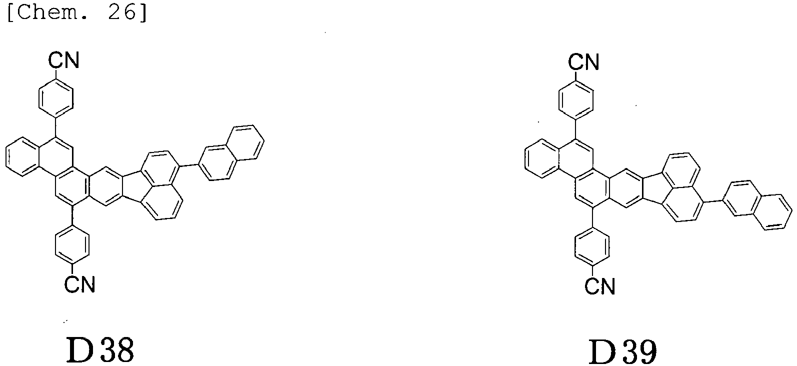

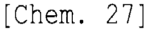

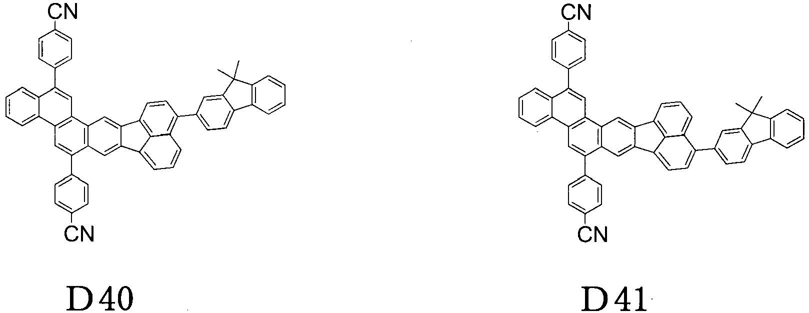

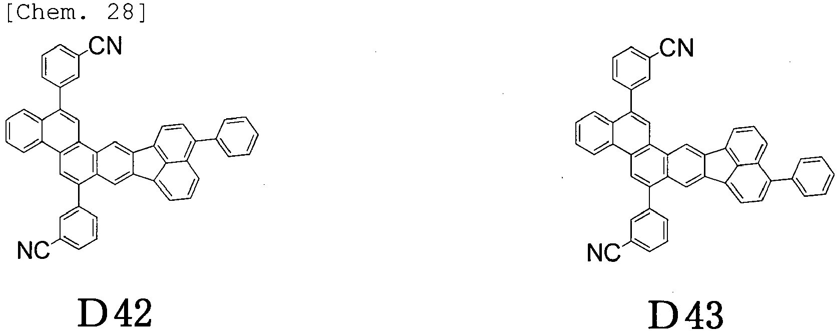

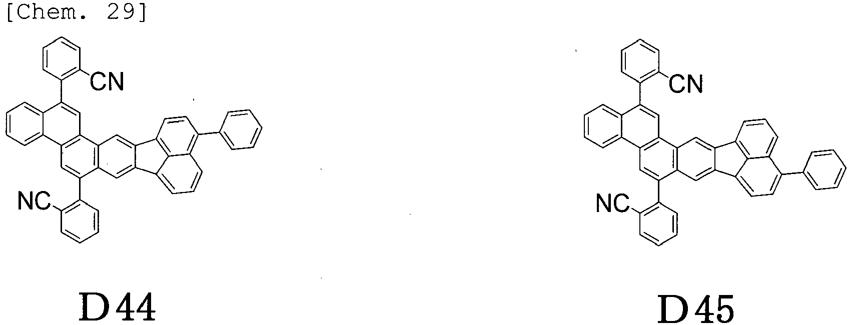

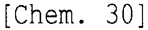

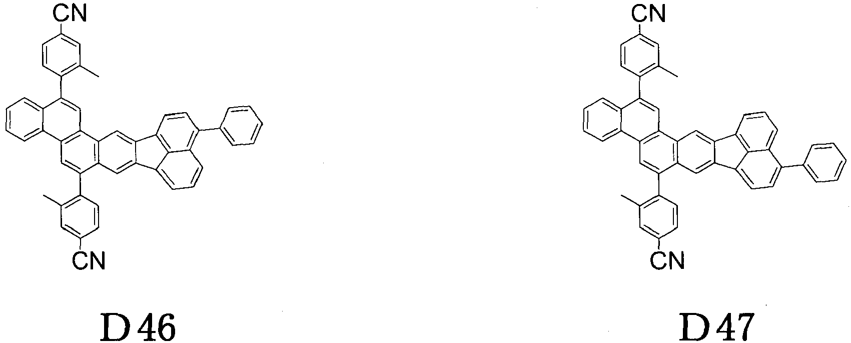

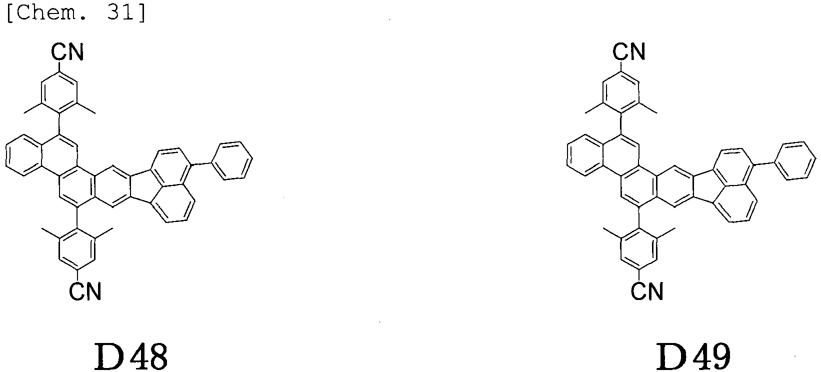

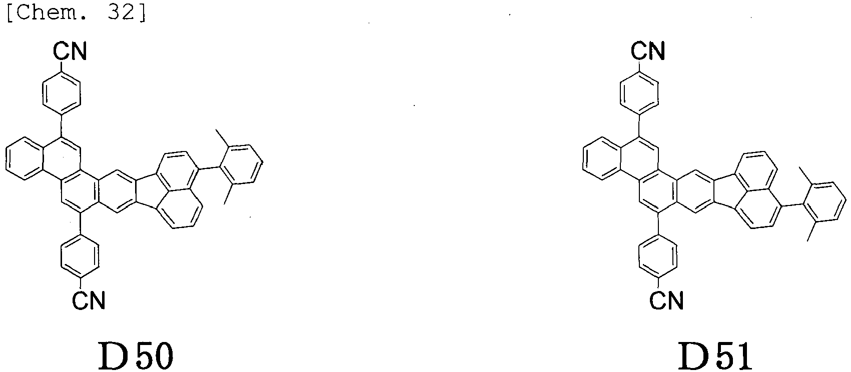

The following Formulae D52 to D69 show examples of the compound represented by Formula [3] or [4], wherein X₃ to X₅ are each an aryl group substituted with a cyano group or a methyl group.

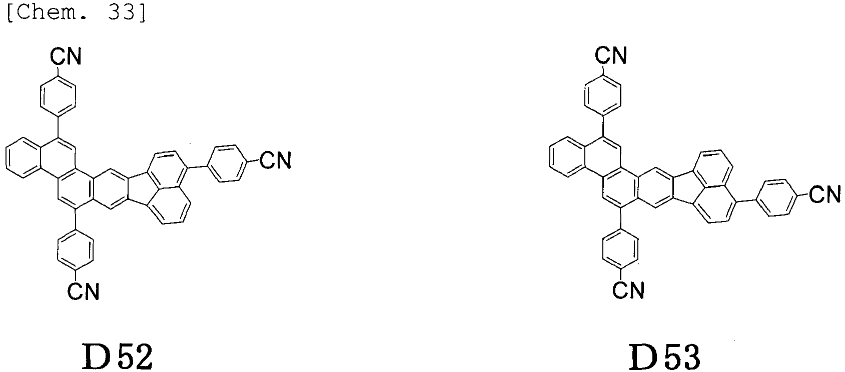

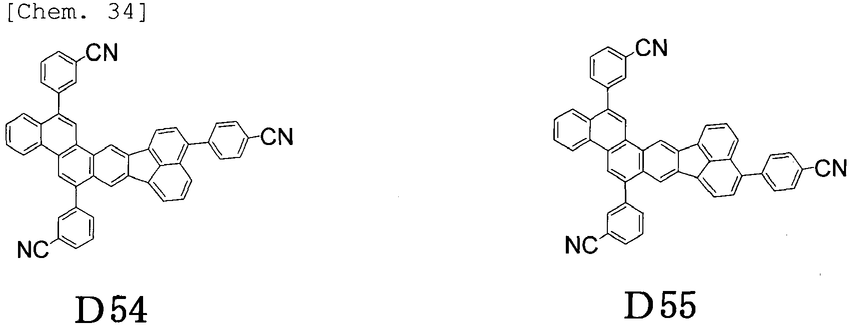

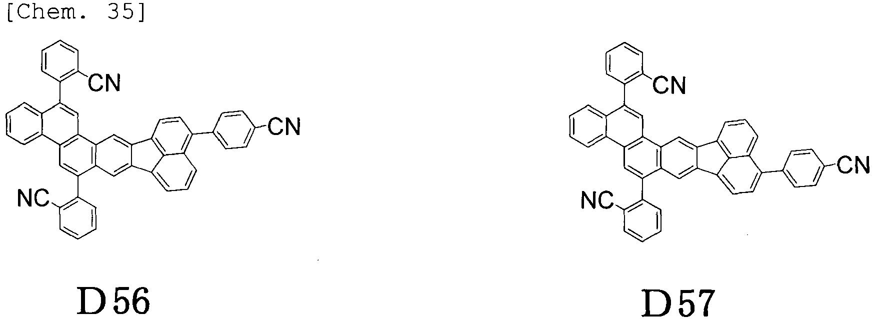

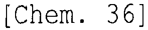

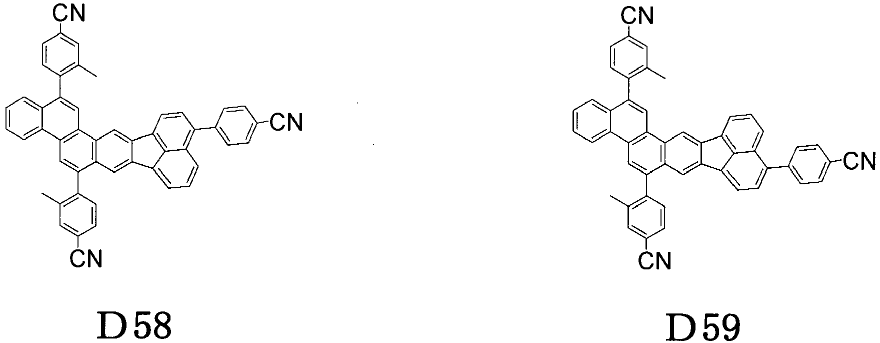

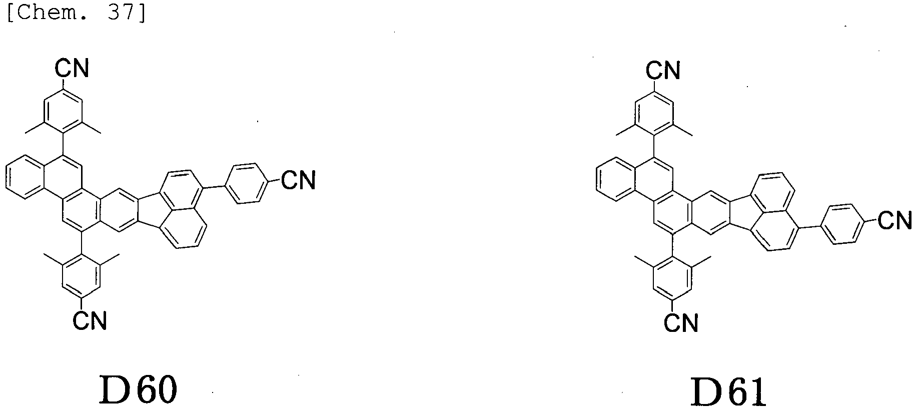

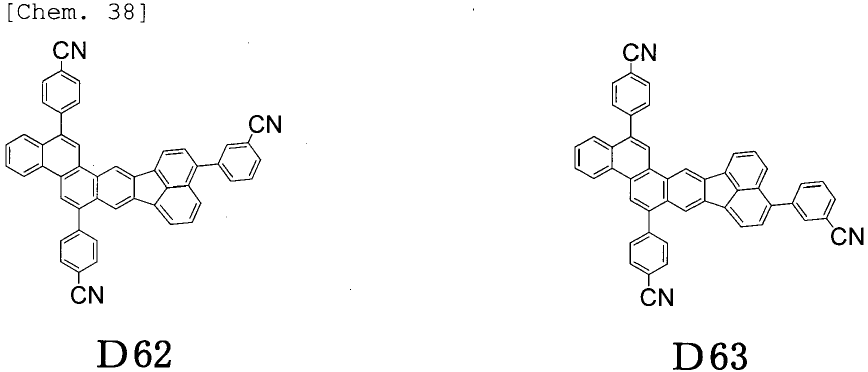

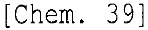

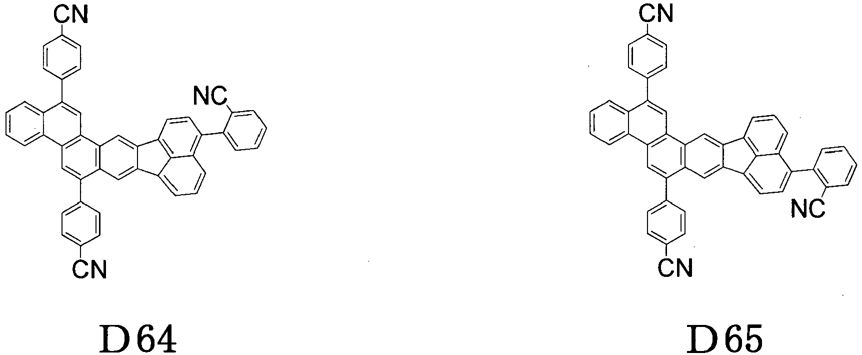

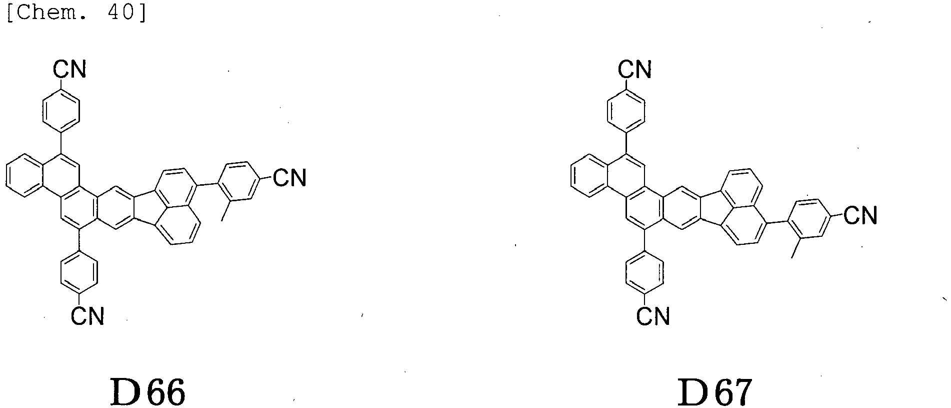

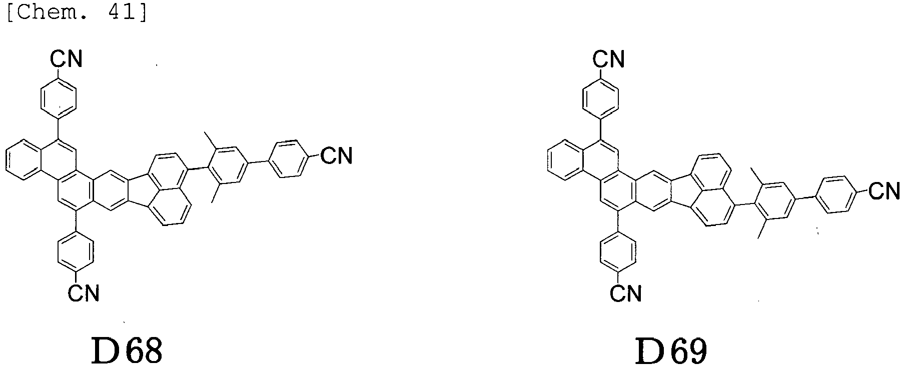

The following Formulae D70 to D81 show examples of the compound represented by Formula [3] or [4], wherein X₃ and X₄ are each an aryl group substituted with a cyano group or a methyl group; and X₅ is a substituted or unsubstituted heterocyclic group.

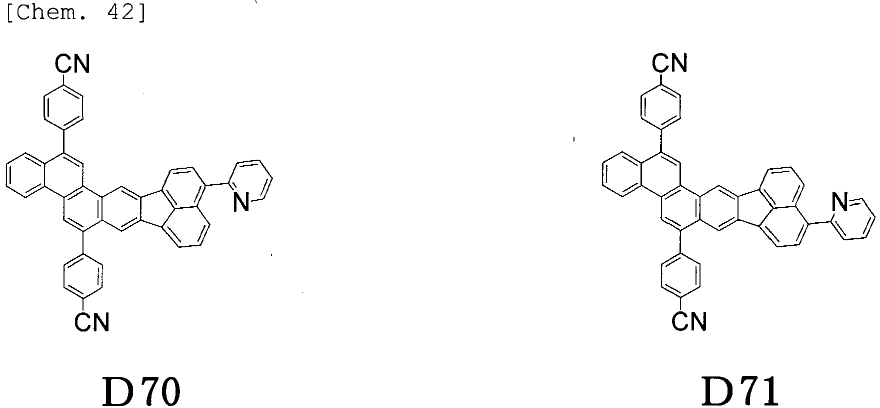

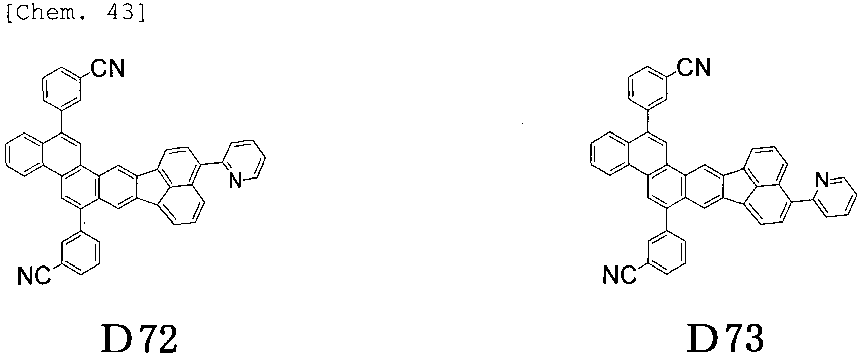

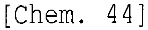

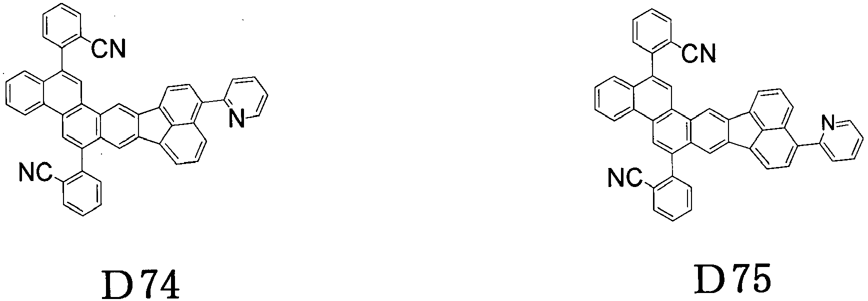

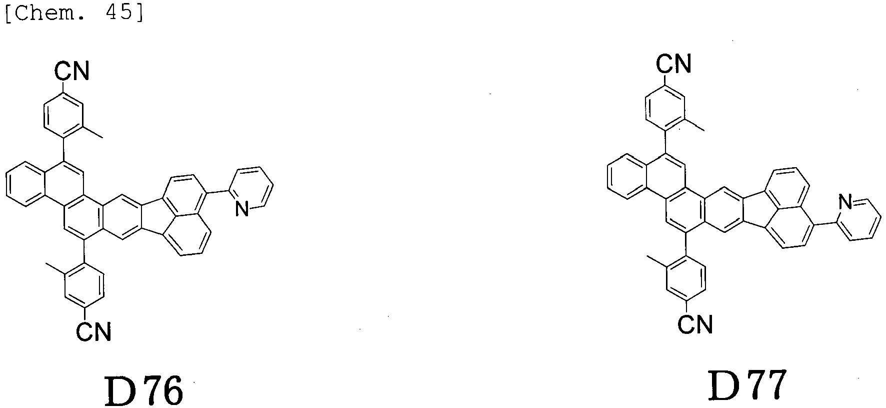

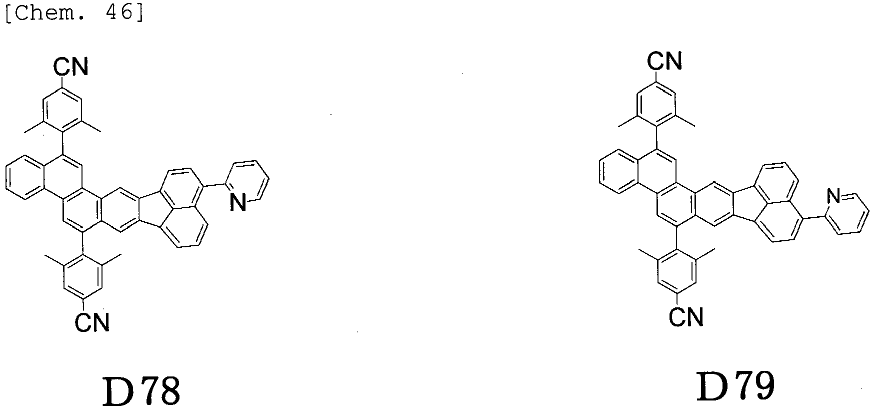

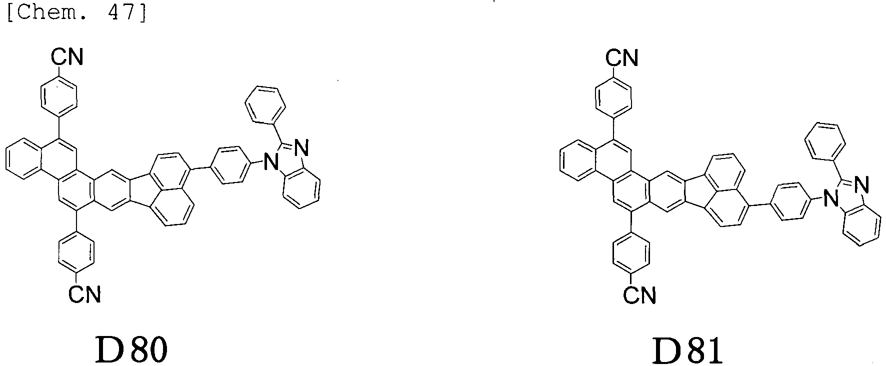

The fused polycyclic compound of the present invention can be a compound represented by the following Formula [5]: 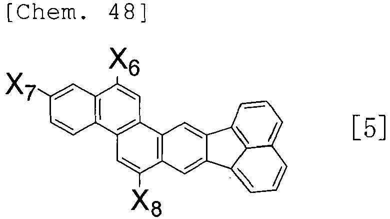 wherein X₆ to X₈ each independently represent a group selected from a cyano group, substituted or unsubstituted alkyl groups, substituted or unsubstituted aryl groups, and substituted or unsubstituted heterocyclic groups.

The substituents optionally possessed by the alkyl groups, the aryl groups, and the heterocyclic groups are the same as those optionally possessed by the substituents in Formula [1].

The following Formulae D82 to D91 show examples of the compound represented by Formula [5], wherein X₆ and X₈ are each an aryl group substituted with a cyano group or a methyl group; and X₇ is an aryl group substituted with a cyano group or a trifluoromethyl group, an alkyl group, or an cyano group.

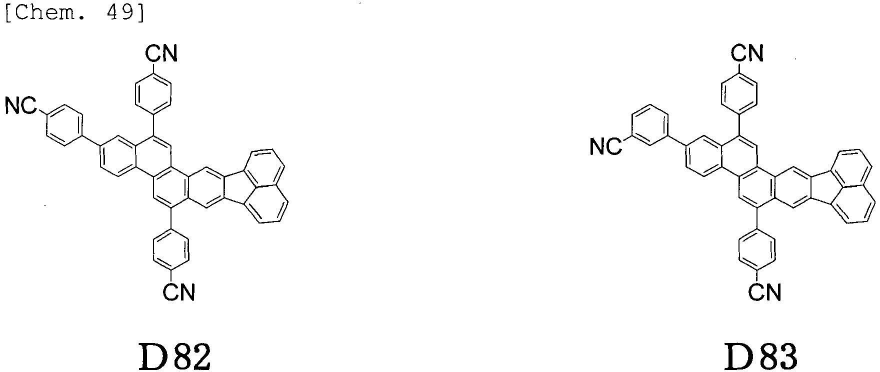

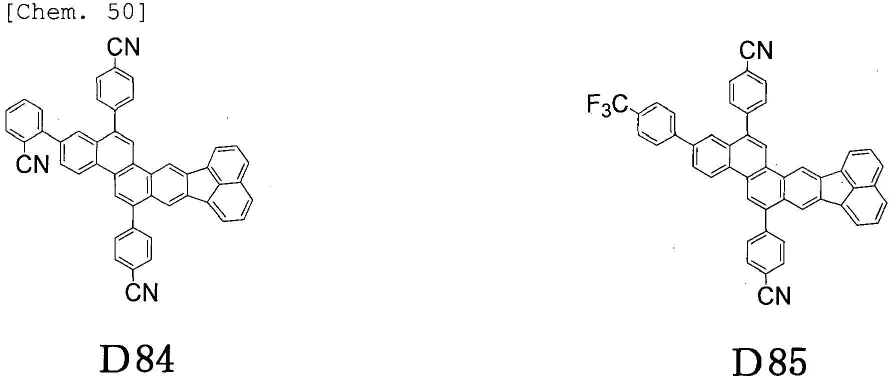

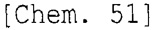

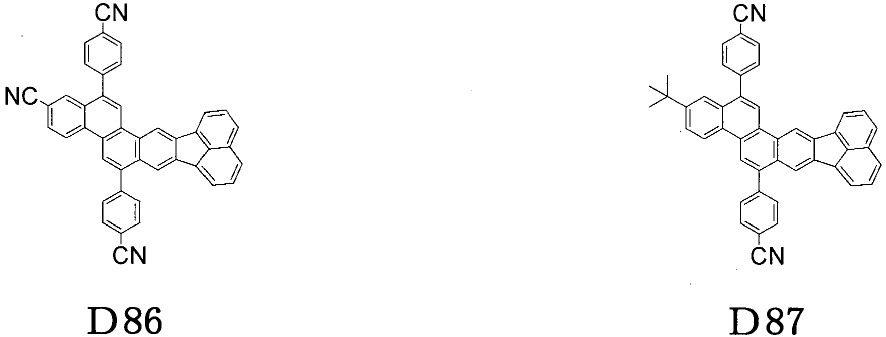

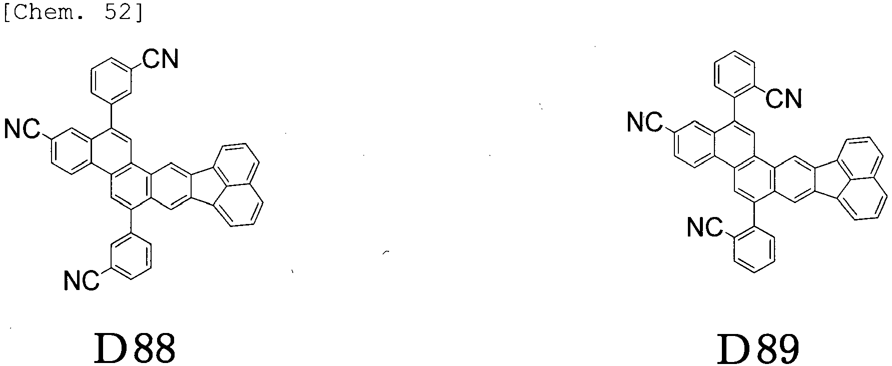

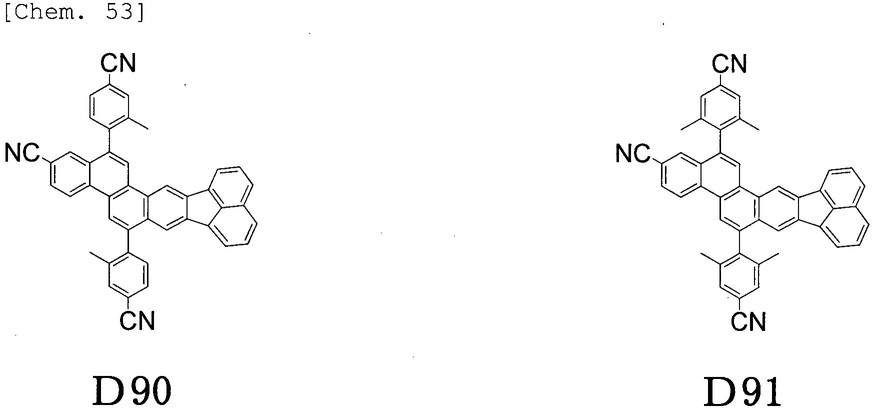

The following Formulae D92 and D93 show examples of the compound represented by Formula [5], wherein X₆ and X₈ are each a heterocyclic group; and X₇ is a cyano group.

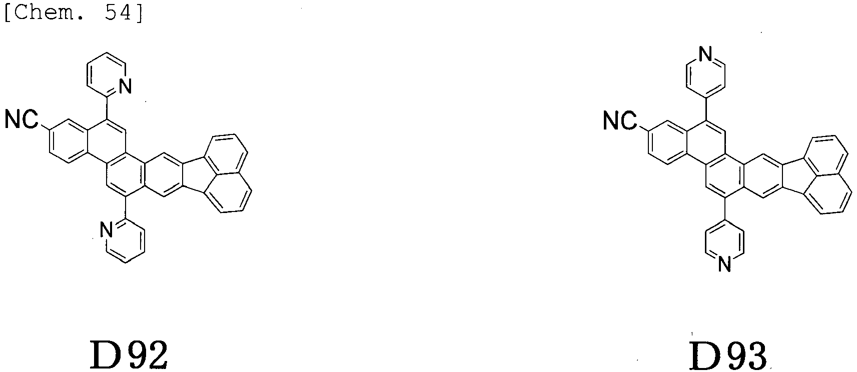

The fused polycyclic compound of the present invention can be a compound represented by the following Formula [6] or [7]: 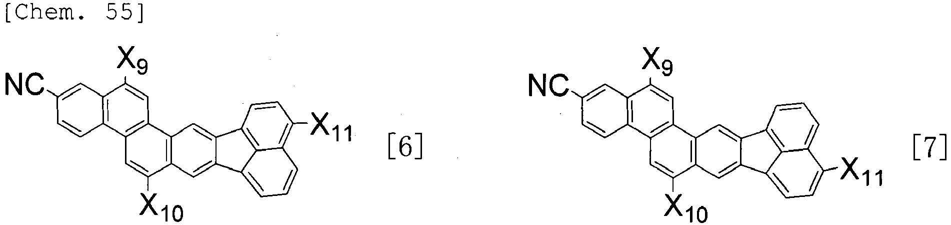 wherein X₉ to X₁₁ each independently represent a group selected from a cyano group, substituted or unsubstituted aryl groups, and substituted or unsubstituted heterocyclic groups.

The substituents optionally possessed by the alkyl groups, the aryl groups, and the heterocyclic groups are the same as those optionally possessed by the substituents in Formula [1].

The following Formulae D94 and D95 show examples of the compound represented by Formula [6] or [7], wherein X₉ and X₁₀ are each an aryl group substituted with a cyano group or a methyl group; and X₁₁ is a cyano group.

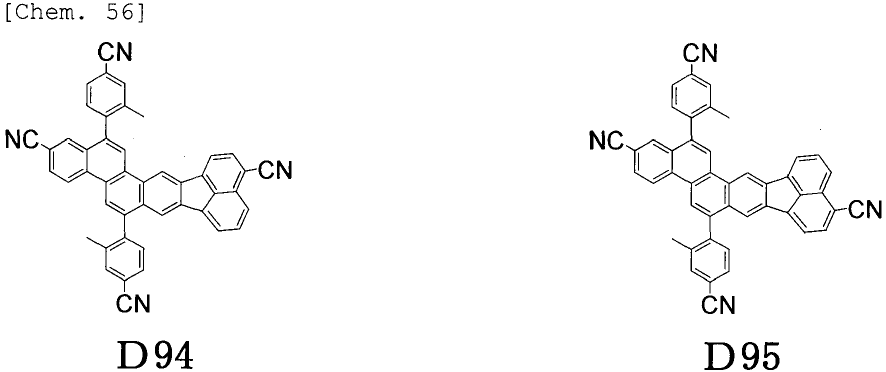

The following Formulae D96 to D103 show examples of the compound represented by Formula [6] or [7], wherein X₉ and X₁₀ are each an aryl group substituted with a cyano group or a methyl group; and X₁₁ is an unsubstituted aryl group or an aryl group substituted with an alkyl group.

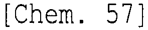

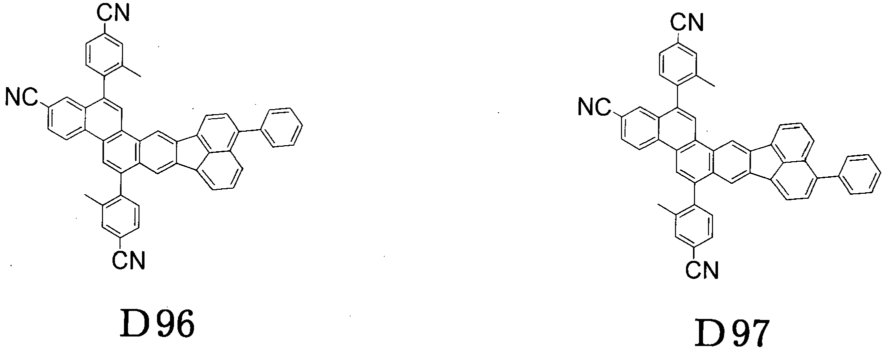

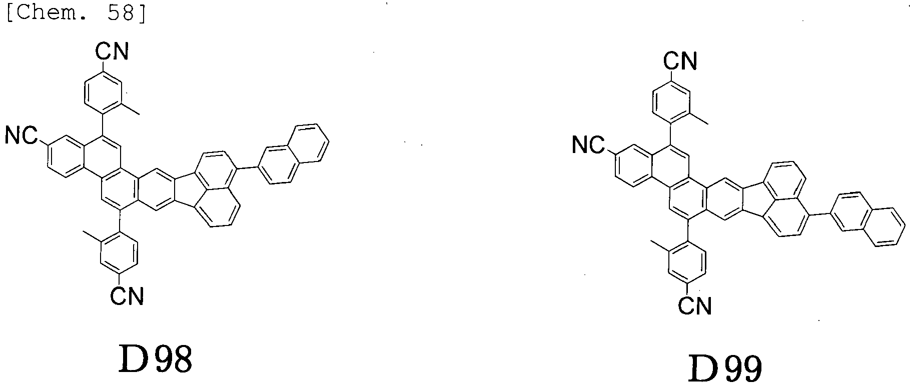

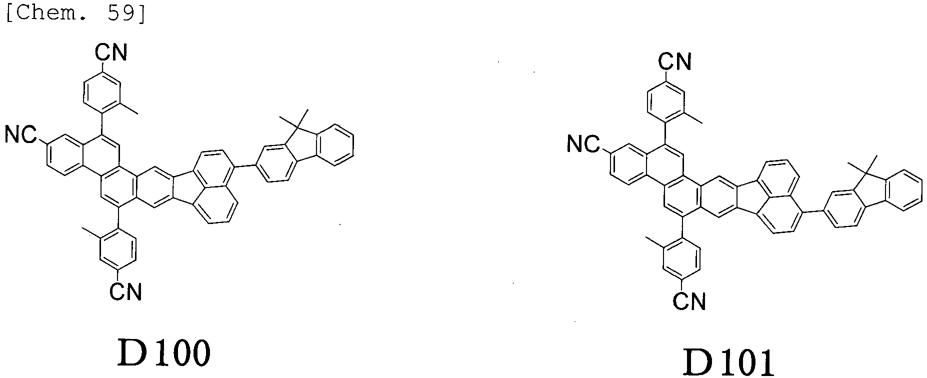

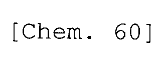

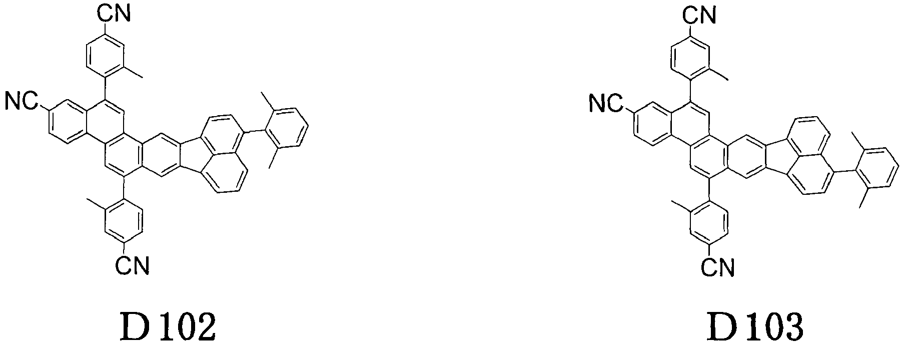

The following Formulae D104 to D115 show examples of the compound represented by Formula [6] or [7], wherein X₉ to X₁₁ are each an aryl group substituted with a cyano group, a methyl group, or a trifluoromethyl methyl group.

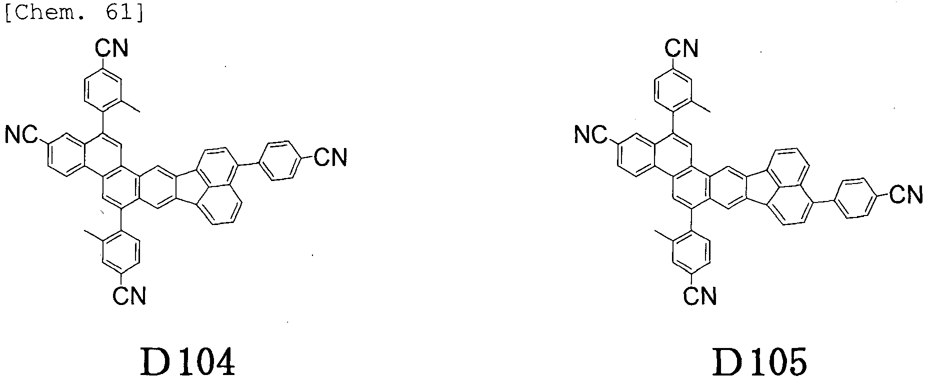

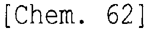

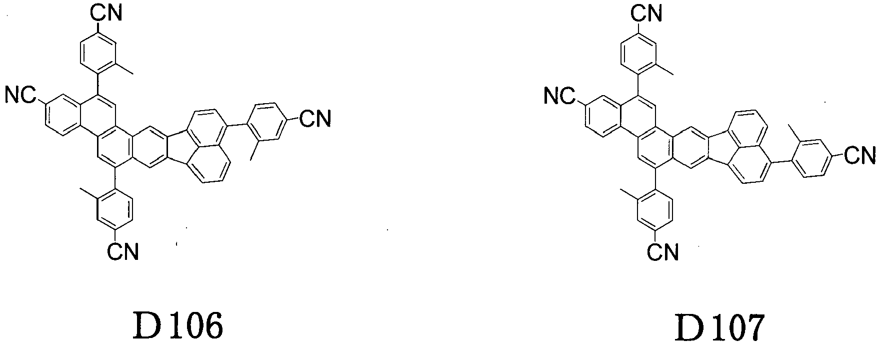

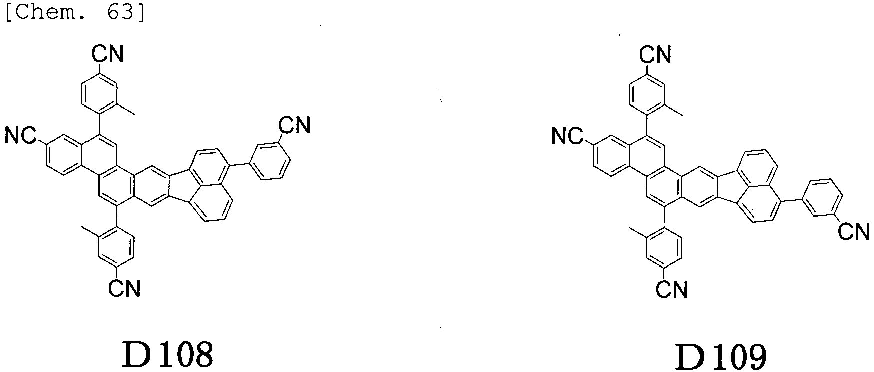

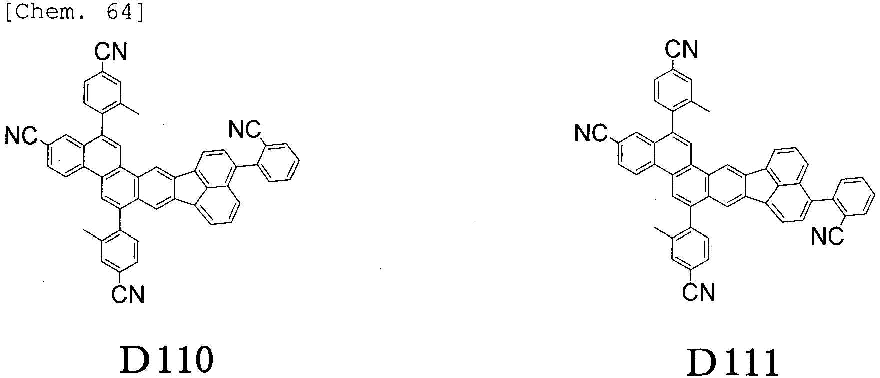

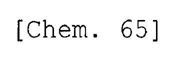

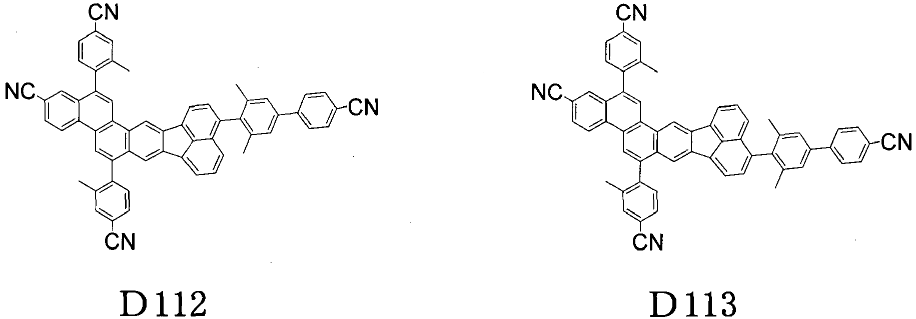

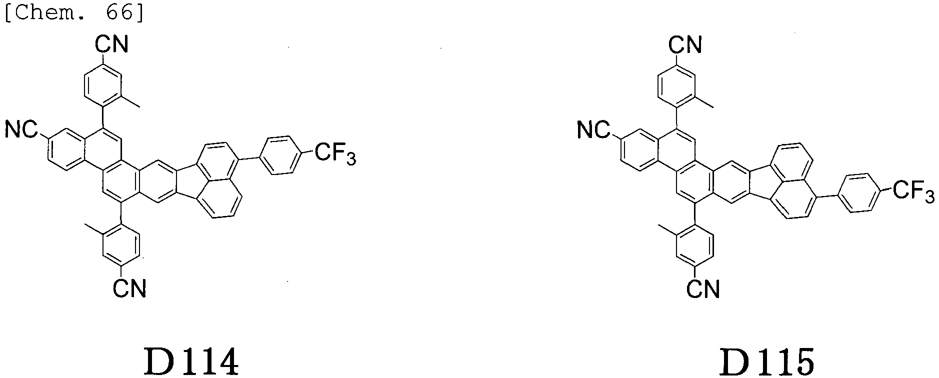

The following Formulae D116 and D117 show examples of the compound represented by Formula [6] or [7], wherein X₉ and X₁₀ are each an aryl group substituted with a cyano group or a methyl group; and X₁₁ is a substituted or unsubstituted heterocyclic group.

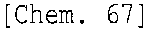

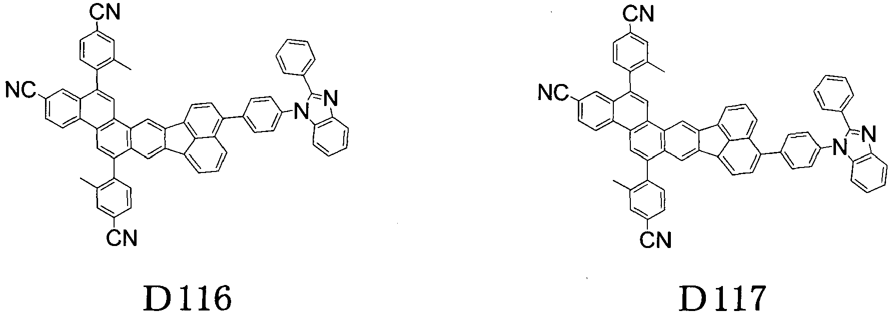

The following Formulae D118 to D121 show examples of the compound represented by Formula [6] or [7], wherein X₉ and X₁₀ are each an unsubstituted aryl group or an aryl group substituted with an alkyl group; and X₁₁ is an aryl group substituted with a cyano group.

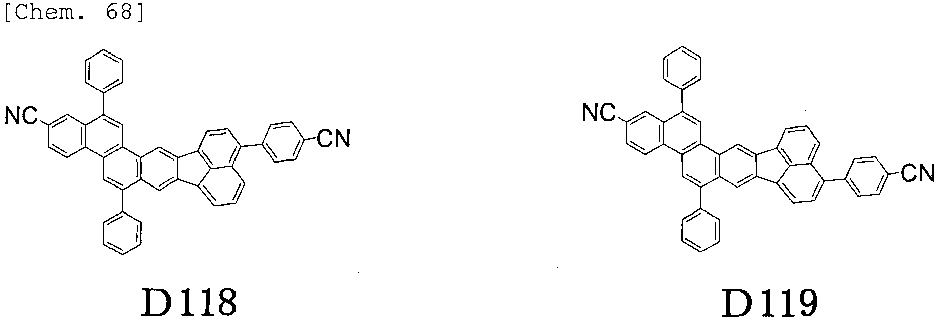

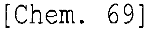

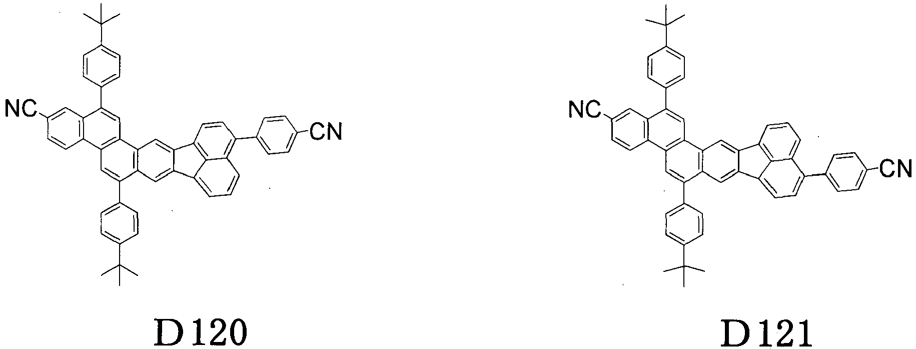

The fused polycyclic compound of the present invention can be a compound represented by the following Formula [8] or [9]: 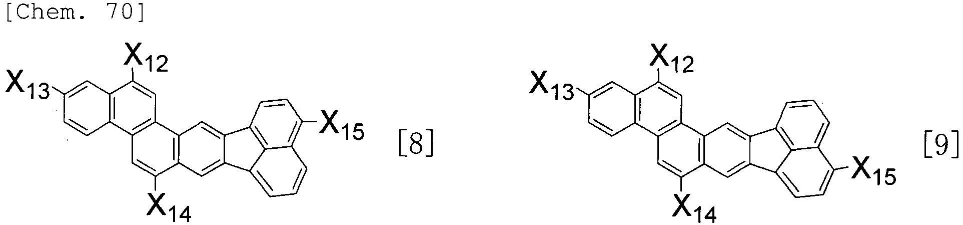 wherein X₁₂ to X₁₅ each independently represent a group selected from substituted or unsubstituted alkyl groups, substituted or unsubstituted aryl groups, and substituted or unsubstituted heterocyclic groups.

The substituents optionally possessed by the alkyl groups, the aryl groups, and the heterocyclic groups are the same as those optionally possessed by the substituents in Formula [1].

The following Formulae D122 to D125 show examples of the compound represented by Formula [8] or [9], wherein X₁₂ to X₁₄ are each an unsubstituted aryl group; and X₁₅ is an alkyl group.

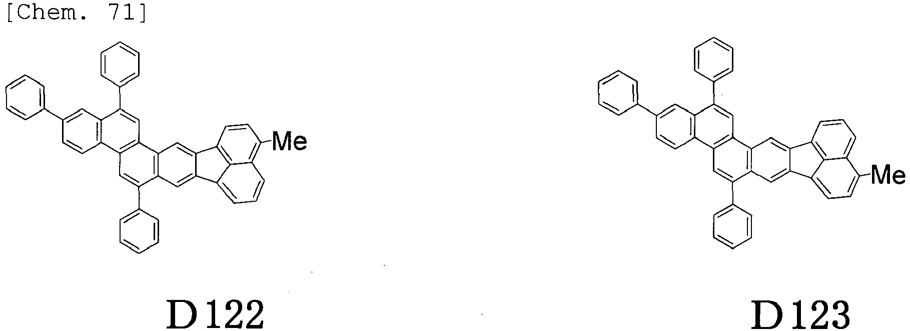

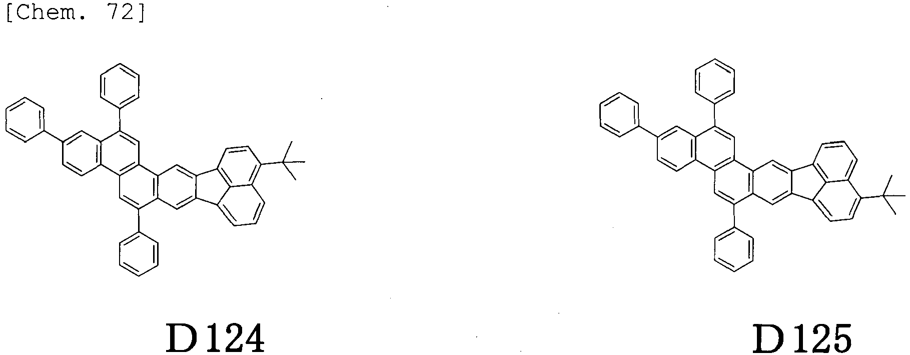

The following Formulae D126 to D129 show examples of the compound represented by Formula [8] or [9], wherein X₁₂ to X₁₄ are each an unsubstituted aryl group or an aryl group substituted with a cyano group; and X₁₅ is a substituted or unsubstituted heterocyclic group.

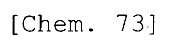

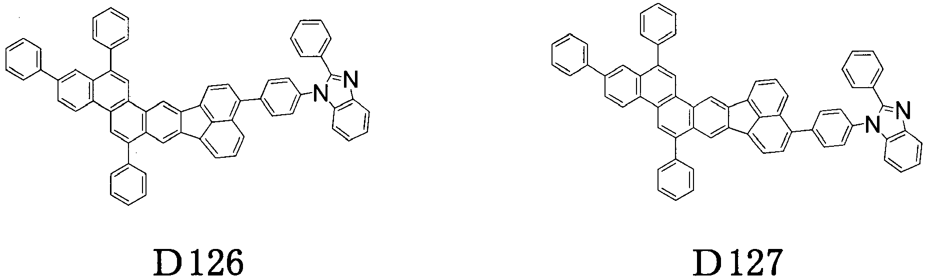

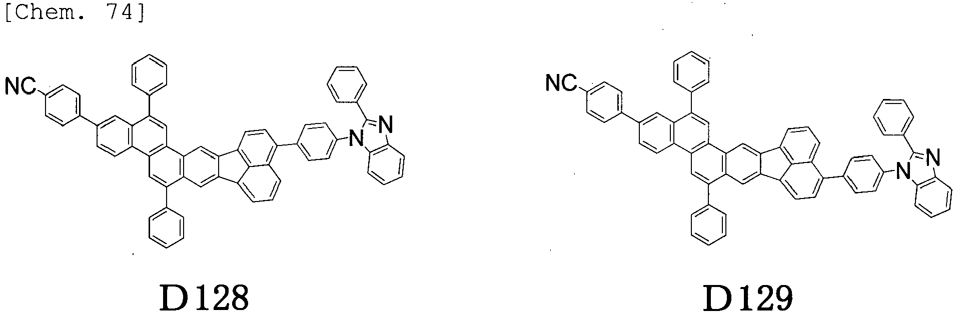

The following Formulae D130 and D131 show examples of the compound represented by Formula [8] or [9], wherein X₁₂ and X₁₄ are each an aryl group substituted with a cyano group or a methyl group; and X₁₃ and X₁₅ are each an unsubstituted aryl group or an unsubstituted heterocyclic group.

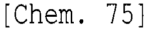

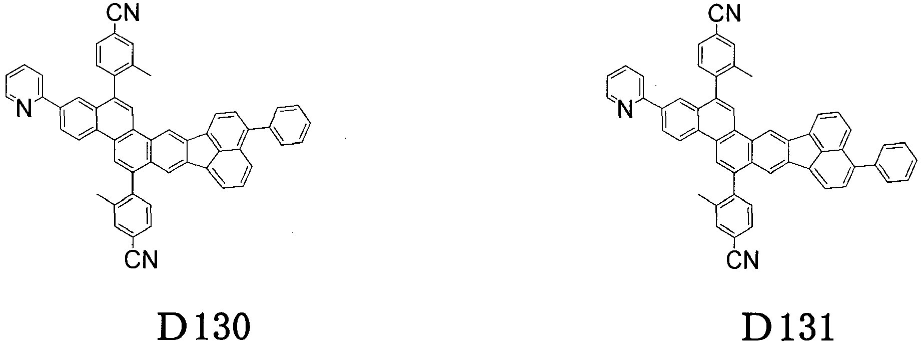

### Description of synthetic route

The fused polycyclic compound represented by Formula [1] can be synthesized as in synthetic routes 1 to 3 shown below.

In synthesis through synthetic route 1 and synthetic route 3, isomers are produced due to the substituent Y₂ or Y₆. The isomers do not substantially differ in the luminescence properties as long as that two Y₂s (or Y₆s) are the same and therefore may be used after isolation by, for example, recrystallization or may be directly used as a mixture.

Since the luminescence properties of the mixture are not low compared to those of a single isomer, the mixture ratio is not specifically limited. In addition, the mixture can suppress crystallization and, thereby, can be expected to show an effect of inhibiting concentration quenching.

### Synthetic route 1

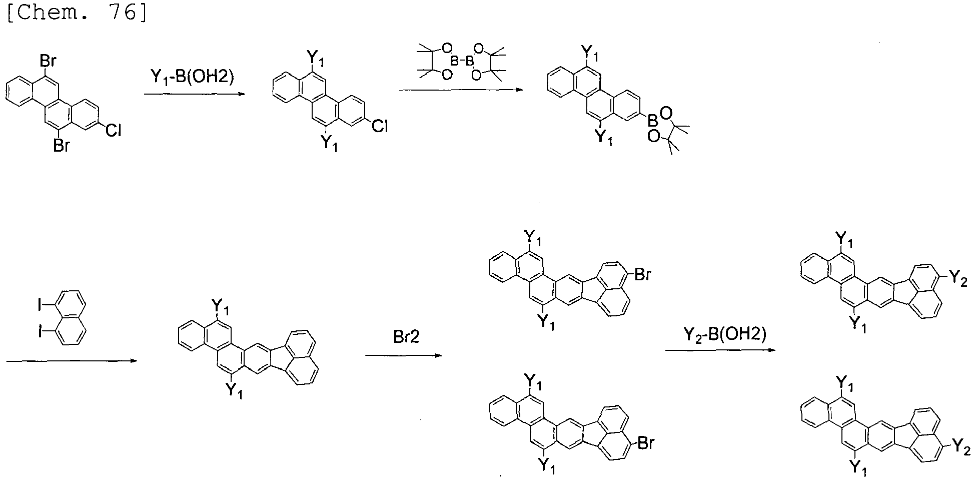

### Synthetic route 2

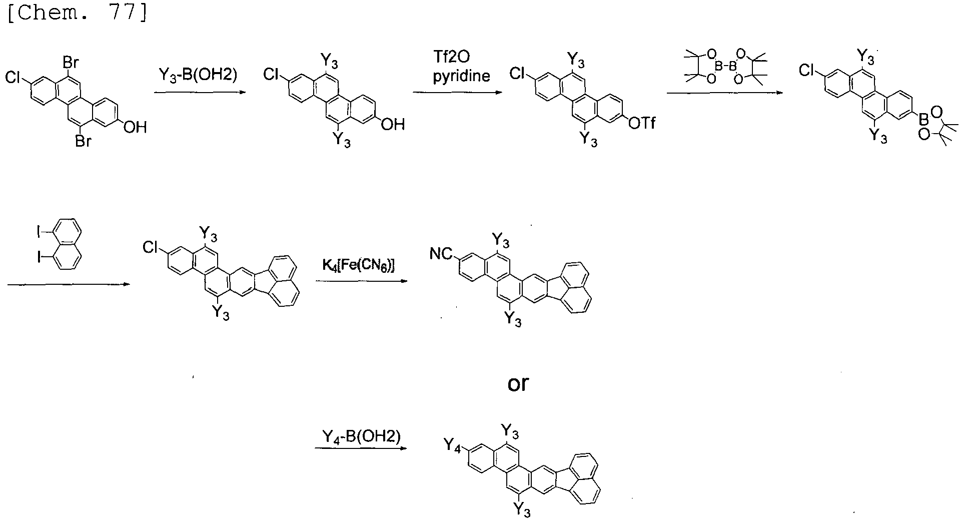

### Synthetic route 3

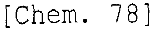

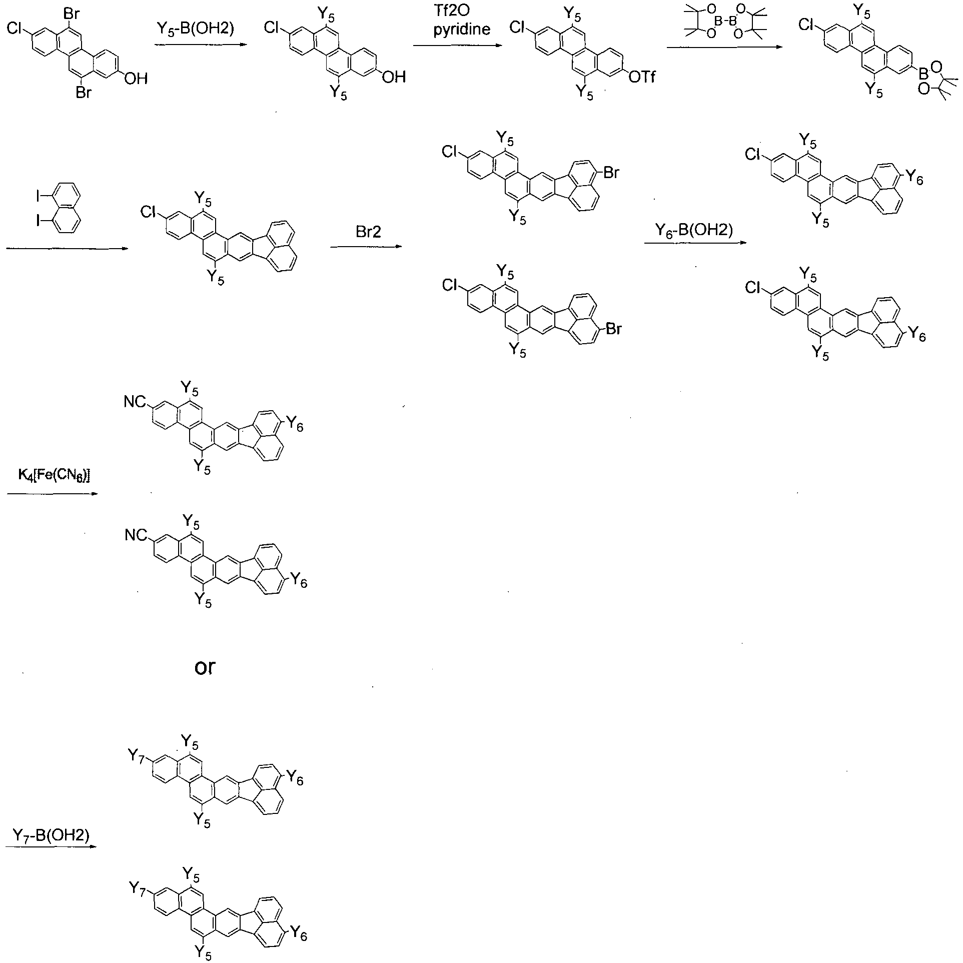

Examples of the organic compound prepared by synthetic route 1 are shown below. Table 2 shows organic compounds having two substituents, and Table 3 shows organic compounds having three substituents.

**[Table 2]**

| | Y1 | Y2 | Synthetic example |
|---|---|---|---|
| Synthetic compound 1 | 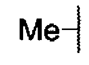 | | 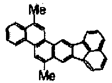 |
| Synthetic compound 2 | 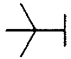 | | 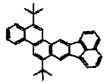 |
| Synthetic compound 3 | 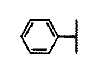 | | 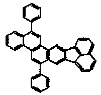 |
| Synthetic compound 4 | 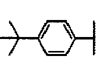 | | 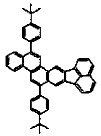 |
| Synthetic compound 5 | 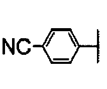 | | 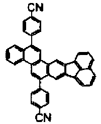 |
| Synthetic compound 6 | 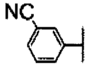 | | 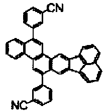 |
| Synthetic compound 7 | 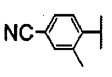 | | 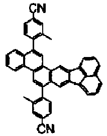 |
| Synthetic compound 8 | 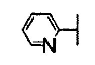 | | 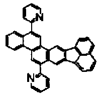 |
| Synthetic compound 9 | 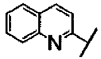 | | 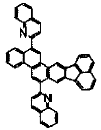 |

**[Table 3]**

| | Y1 | Y2 | Synthetic example | |
|---|---|---|---|---|
| Synthetic compound 10 | 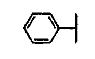 | 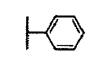 | 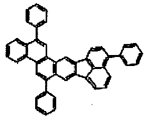 | 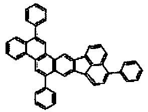 |
| Synthetic compound 11 | 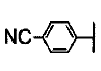 | 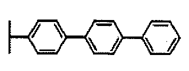 | 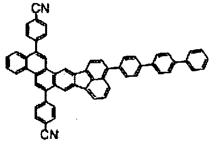 | 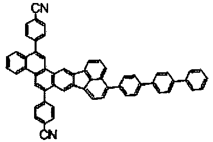 |
| Synthetic compound 12 | 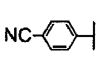 | 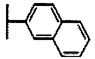 | 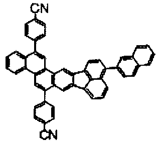 | 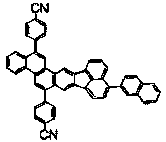 |
| Synthetic compound 13 | 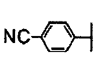 | 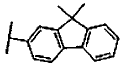 | 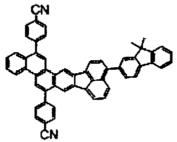 | 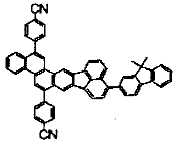 |
| Synthetic compound 14 | 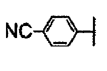 | 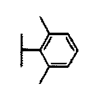 | 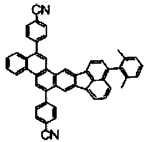 | 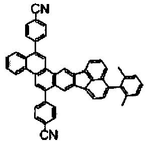 |
| Synthetic compound 15 | 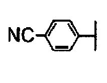 | 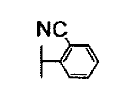 | 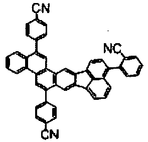 | 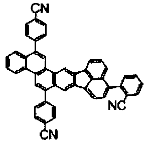 |
| Synthetic compound 16 | 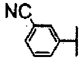 | 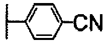 | 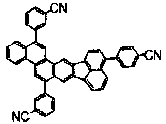 | 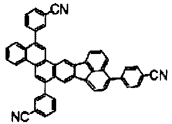 |
| Synthetic compound 17 | 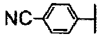 | 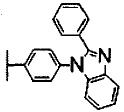 | 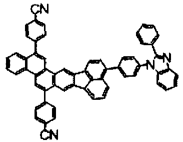 | 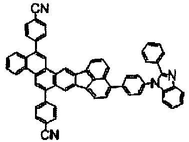 |
| Synthetic compound 18 | 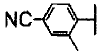 | 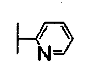 | 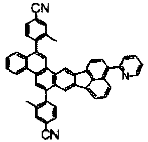 | 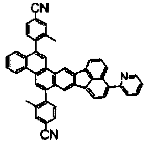 |

Examples of the organic compound prepared by synthetic route 2 are shown in Table 4.

**[Table 41**

| | Y3 | Y4 | Synthetic example |
|---|---|---|---|
| Synthetic compound 19 | 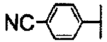 | 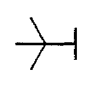 | 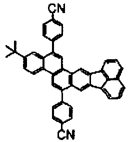 |
| Synthetic compound 20 | 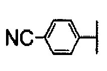 | | 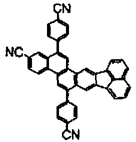 |
| Synthetic compound 21 | 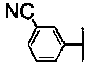 | | 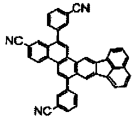 |
| Synthetic compound 22 | 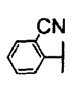 | | 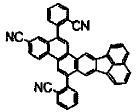 |
| Synthetic compound 23 | 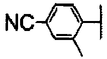 | | 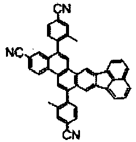 |
| Synthetic compound 24 | 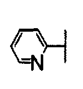 | | 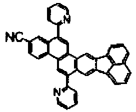 |
| Synthetic compound 25 | 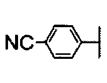 | 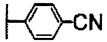 | 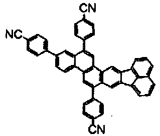 |
| Synthetic compound 26 | 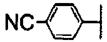 | 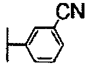 | 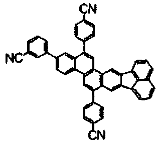 |
| Synthetic compound 27 | 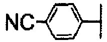 | 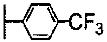 | 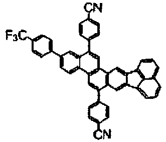 |

Examples of the organic compound prepared by synthetic route 3 are shown in Table 5.

**[Table 5]**

| | Y5 | Y6 | Y7 | Synthetic example | |
|---|---|---|---|---|---|
| Synthetic compound 28 | 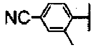 | 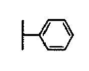 | | 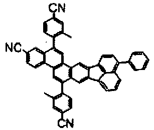 | 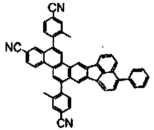 |
| Synthetic compound 29 | 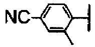 | 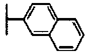 | | 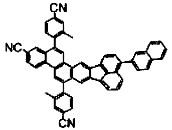 | 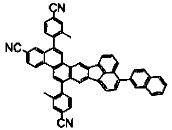 |
| Synthetic compound 30 | 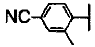 | 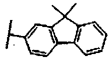 | | 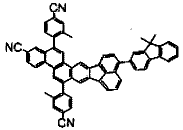 | 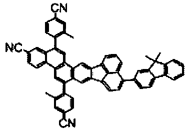 |
| Synthetic compound 31 | 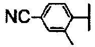 | 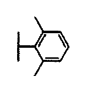 | | 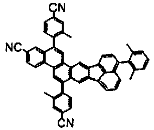 | 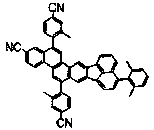 |
| Synthetic compound 32 | 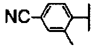 | 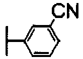 | | 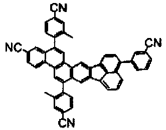 | 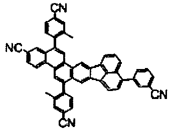 |
| Synthetic compound 33 | 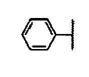 | 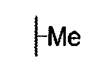 | 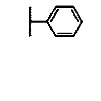 | 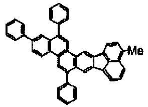 | 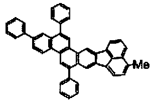 |
| Synthetic compound 34 | 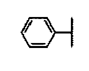 | 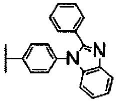 | 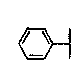 | 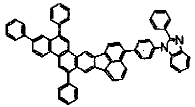 | 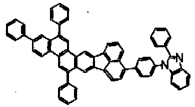 |
| Synthetic compound 35 | 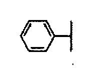 | 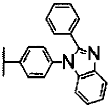 | 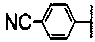 | 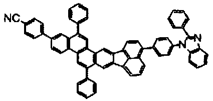 | 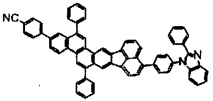 |
| Synthetic compound 36 | 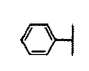 | 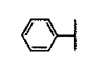 | 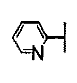 | 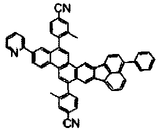 | 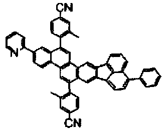 |

### Description of organic light-emitting device in the embodiment

An organic light-emitting device according to the embodiment will be described.

The organic light-emitting device according to the embodiment includes a pair of electrodes, i.e., an anode and a cathode, and an organic compound layer disposed between the electrodes. The organic compound layer is an element including the organic compound represented by Formula [1].

The organic compound layer of the organic light-emitting device according to the embodiment may have a monolayer structure or a multi-layer structure.

Here, the multi-layer is a layer appropriately selected from the group consisting of hole-injecting layers, hole-transporting layers, light-emitting layers, hole-blocking layers, electron-transporting layers, electron-injecting layers, and exciton-blocking layers and may be a combination of layers selected from the group.

The configuration of the organic light-emitting device according to the embodiment is not limited thereto, and various layer configurations can be employed. For example, an insulating layer may be disposed at the interface between an electrode and an organic compound layer; an adhesive layer or an interference layer may be provided; or an electron-transporting layer or a hole-transporting layer may be composed of two layers having different ionization potentials.

The configuration of the device may be a top emission system, which extracts light from the electrode on the substrate side, or a bottom emission system, which extracts light from the opposite side of the substrate. Alternatively, a configuration in which light is extracted from both sides can also be employed.

In the organic light-emitting device according to the embodiment, the light-emitting layer can contain the organic compound of the present invention.

The concentration of the host material in the light-emitting layer of the organic light-emitting device according to the embodiment is 50 wt% or more and 99.9 wt% or less, in particular, 80 wt% or more and 99.5 wt% or less, based on the total amount of the light-emitting layer.

The concentration of the guest material in the light-emitting layer of the organic light-emitting device according to the embodiment is 0.1 wt% or more and 30 wt% or less, in particular, 0.5 wt% or more and 10 wt% or less, based on the amount of the host material. The light-emitting layer may have a monolayer structure or a multi-layer structure.

A plurality of the light-emitting layers may be stacked or may be horizontally arranged. In the horizontal arrangement, every light-emitting layer is in contact with the adjacent layers such as a hole-transporting layer and an electron-transporting layer.

The light-emitting layer may have a configuration where a single light-emitting layer contains light-emitting materials emitting light of different colors. In such a case, the light-emitting materials may form the respective domains.

The organic light-emitting device according to the embodiment may emit white light by containing different light-emitting materials in the light-emitting layer.

In the embodiment, the host material is a compound having a largest weight ratio among the compounds constituting the light-emitting layer, and the guest material is a compound having a smaller weight ratio than that of the host material among the compounds constituting the light-emitting layer and bearing main light emission. The guest material is also called a dopant.

The assist material is a compound having a smaller weight ratio than that of the host material among the compounds constituting the light-emitting layer and assisting the light emission of the guest material. The assist material is also called a second host material.

In the organic light-emitting device according to the embodiment, in addition to the compound according to the present invention, for example, a known hole-injecting material, hole-transporting material, host material, guest material, electron-injecting material, or electron-transporting material can be optionally used. These materials may be a low-molecular compound or a high-molecular compound.

Examples of these compounds are shown below.

As the hole-injecting or transporting material, a material having high hole mobility can be used. Examples of the low- or high-molecular material having hole-injecting or transporting ability include, but not limited to, triarylamine derivatives, phenylenediamine derivatives, stilbene derivatives, phthalocyanine derivatives, porphyrin derivatives, poly(vinylcarbazole), poly(thiophene), and other electrically conductive polymers.

Examples of the host material include, but not limited to, triarylamine derivatives, phenylene derivatives, fused ring aromatic compounds (e.g., naphthalene derivatives, phenanthrene derivatives, fluorene derivatives, and chrysene derivatives), organic metal complexes (e.g., organic aluminum complexes such as tris(8-quinolinolate)aluminum, organic beryllium complexes, organic iridium complexes, and organic platinum complexes), and polymer derivatives such as poly(phenylenevinylene) derivatives, poly(fluorene) derivatives, poly(phenylene) derivatives, poly(thienylenevinylene) derivatives, and poly(acetylene) derivatives.

The electron-injecting or transporting material is appropriately selected by considering the balance with the hole mobility of the hole-injecting or transporting material.

Examples of the material having electron-injecting or transporting ability include, but not limited to, oxadiazole derivatives, oxazole derivatives, pyrazine derivatives, triazole derivatives, triazine derivatives, quinoline derivatives, quinoxaline derivatives, phenanthroline derivatives, and organic aluminum complexes.

As the anode material, a material having a higher work function is used. Examples of the material include simple metals such as gold, platinum, silver, copper, nickel, palladium, cobalt, selenium, vanadium, and tungsten; alloys of these simple metals; and metal oxides such as tin oxide, zinc oxide, indium oxide, indium tin oxide (ITO), and indium zinc oxide. In addition, electrically conductive polymers such as polyaniline, polypyrrole, and polythiophene can be used.

These electrode materials may be used alone or in combination. The anode may have a monolayer structure or a multi-layer structure.

In contrast, as the cathode material, a material having a lower work function is used. Examples of the material include alkali metals such as lithium; alkaline earth metals such as calcium; simple metals such as aluminum, titanium, manganese, silver, lead, and chromium; and alloys of these simple metals, such as magnesium-silver, aluminum-lithium, and aluminum-magnesium. In addition, metal oxides such as indium tin oxide (ITO) can be used. These electrode materials may be used alone or in combination. The cathode may have a monolayer structure or a multi-layer structure.

In the organic light-emitting device according to the embodiment, a layer containing the organic compound according to the embodiment and layers of other organic compounds are formed by the following methods.

For example, a layer is formed by vacuum vapor deposition, ionized vapor deposition, sputtering, plasma coating, or known coating such as spin coating, dipping, a casting method, an LB method, or an ink-jetting method of a compound dissolved in an appropriate solvent.

In the case of forming a layer by vacuum deposition or solution coating, crystallization hardly occurs, and the resulting layer shows excellent stability for a long time. In addition, in coating, a film can be formed in combination with an appropriate binder resin.

Examples of the binder resin include, but not limited to, polyvinyl carbazole resins, polycarbonate resins, polyester resins, ABS resins, acrylic resins, polyimide resins, phenol resins, epoxy resins, silicone resins, and urea resins.

These binder resins may be used alone as a homopolymer or a copolymer or in combination of two or more thereof. In addition, known additives such as a plasticizer, an antioxidant, and a UV absorber may be optionally used together with the binder resin.

### Use of organic light-emitting device according to the embodiment

The organic light-emitting device according to the embodiment can be used as a component of a display apparatus or a lighting system. Other examples of the use include exposure light sources of electrophotographic image forming apparatuses, backlights of liquid crystal display apparatuses, and light sources. The organic light-emitting device may further include a color filter.

The display apparatus according to the embodiment has a display section including a plurality of pixels.

The pixels each include the organic light-emitting device according to the embodiment and an active device. An example of the active device is a switching device for controlling luminance. An example of the switching device is a TFT device.

The anode or the cathode of the organic light-emitting device is connected to the drain electrode or the source electrode of the TFT device. The display apparatus can be used as an image display apparatus of a personal computer (PC). The TFT device is disposed on the insulative surface of the substrate.

The display apparatus may be an image information processing apparatus that includes an image input section for inputting image information from an area CCD, a linear CCD, or a memory card and displays the input image on a display section.

The display section of the image information processing apparatus or the image forming apparatus may have a touch panel function. The display apparatus may be used in the display section of a multi-functional printer.

The lighting system is an apparatus for lighting, for example, a room. The lighting system may emit light of white or neutral white or light of a wavelength of blue or red light.

The lighting system according to the embodiment includes the organic light-emitting device according to the embodiment and an AC/DC converter circuit connected to the organic light-emitting device. The lighting system may include a color filter.

The AC/DC converter circuit according to the embodiment converts AC voltage to DC voltage.

The image forming apparatus according to the embodiment includes a photosensitive member, a charging unit for charging a surface of the photosensitive member, an exposure unit for forming an electrostatic latent image by exposing the photosensitive member to light, and a developing unit for developing the electrostatic latent image formed on the surface of the photosensitive member. The exposure unit includes the organic light-emitting device of the embodiment.

An example of the exposure unit is an exposure machine including the organic light-emitting device according to the embodiment. In the exposure machine, the organic light-emitting devices may be arranged in lines. Alternatively, the exposure machine may have a configuration such that the entire exposure side emits light.

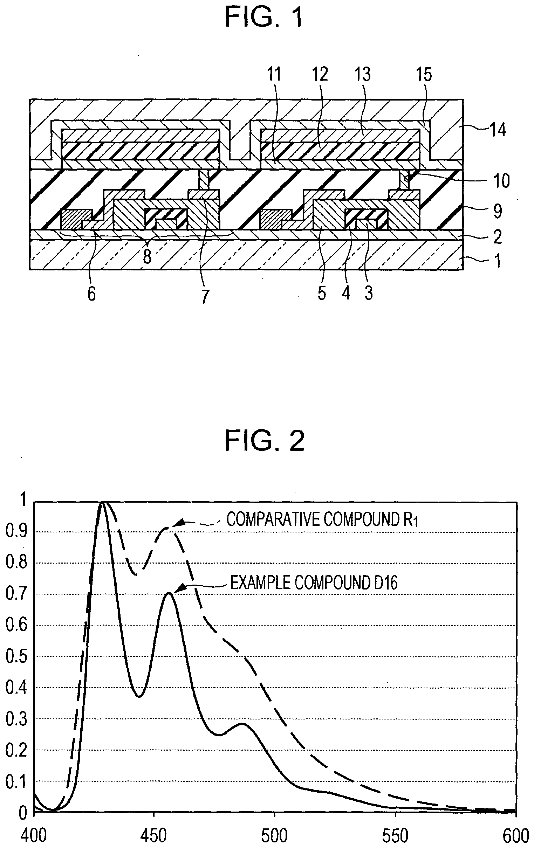

A display apparatus including the organic light-emitting device according to the embodiment will now be described with reference to Fig. 1.

Fig. 1 is a schematic cross-sectional view of a display apparatus having organic light-emitting devices according to the embodiment and TFT devices serving as switching devices connected to the organic light-emitting devices. Fig. 1 shows two pairs of the organic light-emitting device and the TFT device. The structure will now be described in detail.

This display apparatus include a substrate 1 such as a glass substrate and a moisture-proof film 2 disposed above the substrate 1 for protecting the TFT devices or the organic compound layer. Reference numeral 3 denotes a metal gate electrode, reference numeral 4 denotes a gate insulating film, and reference numeral 5 denotes a semiconductor layer.

The TFT device 8 includes a semiconductor layer 5, a drain electrode 6, and a source electrode 7. An insulating film 9 is disposed above the TFT device 8. The anode 11 of the organic light-emitting device and the source electrode 7 are connected to each other via a contact hole 10. The display apparatus is not limited to this configuration as long as either the anode or the cathode is connected to either the source electrode or the drain electrode of the TFT device.

In Fig. 1, the multi-layer organic compound layer 12 is shown as one layer. Furthermore, a first protective layer 14 and a second protective layer 16 are disposed on the cathode 13 for preventing the organic light-emitting device from deteriorating.

The display apparatus according to the embodiment can also use an MIM device as a switching device, instead of the transistor.

The active device of the organic light-emitting device according to the embodiment may be directly formed on a substrate such as a Si substrate. Direct formation on a substrate means that a transistor is formed by machining a substrate itself such as a Si substrate.

Such a configuration is selected depending on the resolution. For example, in a resolution of about 1-inch QVGA, the active devices are directly formed on a Si substrate. The directly formed active devices can be transistors.

It is possible to display high-quality images stably for a long time by driving the display apparatus including the organic light-emitting device according to the embodiment.

The acenaphtho[1,2-b]chrysene compound of the present invention can also be used in an in vivo label or a filter film, in addition to the organic light-emitting device.

### Examples

### Example 1

### Synthesis of Example Compound D16

Example Compound D16 according to the embodiment was synthesized by the following method.

### (1) Synthesis of intermediate compound M1

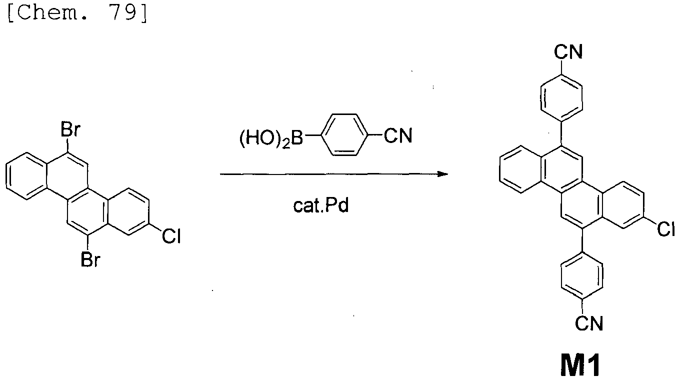

Under a nitrogen atmosphere, 45.0 g (107 mmol) of 6,12-dibromo-2-chrysene, 33.0 g (225 mmol) of 4-cyanophenylboranic acid, 68.1 g (642 mmol) of sodium carbonate, 2700 mL of toluene, 450 mL of ethanol, 450 mL of water, and 3.71 g (3.21 mmol) of tetrakis(triphenylphosphine)palladium were sequentially placed in a 5-L separable flask and were stirred under reflux at 70°C for 5 hours.

The mixture was cooled to room temperature and was then filtered. The resulting solid residue was washed with water and acetone, and the crystals were then dried under reduced pressure to give 43.5 g (yield: 87.3 %) of intermediate compound M1.

### (2) Synthesis of intermediate compound M2

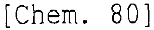

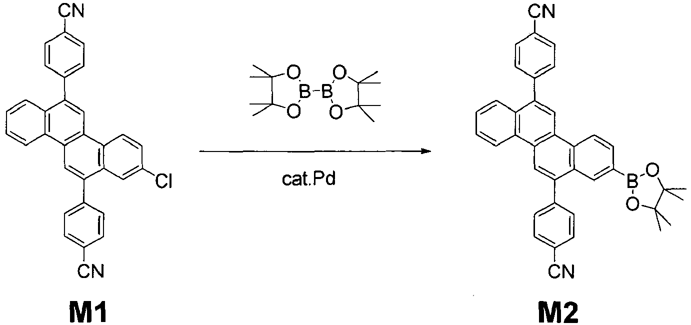

Under a nitrogen atmosphere, 42.8 g (92.1 mmol) of intermediate compound M1, 46.8 g (184 mmol) of bis-dioxaborolane, 27.1 g (276 mmol) of potassium acetate, 1.59 g (2.76 mmol) of bis(dibenzylideneacetone)palladium, and 2.72 g (6.63 mmol) of 2-dicyclohexylphosphino-2',6'-dimethoxybiphenyl were added to 4400 mL of 1,4-dioxane in a 8-L separable flask. The mixture was heated to 100°C and was then stirred at the same temperature for 10 hours.

The mixture was cooled to room temperature and 4400 mL of water added thereto. The mixture was stirred for 2 hours and was then filtered. The resulting solid residue was slurry-washed with water and methanol to give crude crystals of intermediate compound M2.

The resulting crude crystals were dissolved in 4500 mL of chlorobenzene. The solution was purified by column chromatography (chlorobenzene/ethyl acetate = 100/1), followed by slurry-washing with hot toluene. The resulting crystals were dried under reduced pressure to give 32.2 g (yield: 62.9%) of intermediate compound M2 as white crystals.

### (3) Synthesis of intermediate compound M3

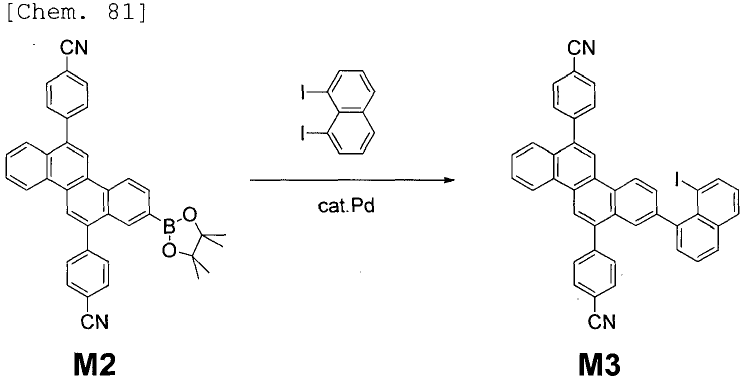

Under a nitrogen atmosphere, 11.3 g (20.3 mmol) of intermediate compound M2, 9.3 g (24.4 mmol) of 1,8-dinaphthalene, 8.6 g (81.2 mmol) of sodium carbonate, and 1.17 g (1.02 mmol) of tetrakis(triphenylphosphine)palladium were added to 1130 mL of DMSO in a 3-L three-neck flask. The mixture was heated to 80°C and was then stirred at the same temperature for 2 hours.

The mixture was cooled to 60°C and 1130 mL of water added thereto. The mixture was stirred for 1 hour and was then filtered. The resulting solid residue was slurry-washed with water and methanol to give crude crystals of intermediate compound M3.

The resulting crude crystals were dissolved in 3840 mL of chlorobenzene. The solution was purified by column chromatography (chlorobenzene), followed by slurry-washing with hot toluene. The resulting crystals were dried under reduced pressure to give 9.50 g (yield: 78.2%) of intermediate compound M3 as yellow crystals.

### (4) Synthesis of Example Compound D16

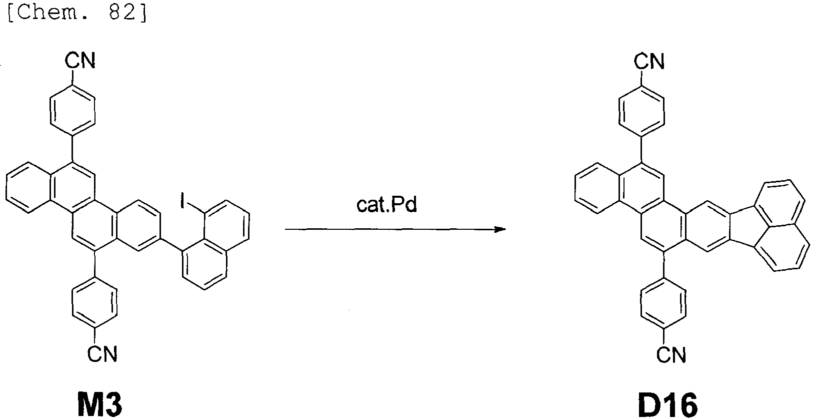

Under a nitrogen atmosphere, 9.40 g (13.8 mmol) of intermediate compound M3, 6.4 g (41.3 mmol) of 1,8-diazabicyclo[5.4.0]-7-undecene, and 0.80 g (0.68 mmol) of tetrakis(triphenylphosphine)palladium were added to 1000 mL of N,N-dimethylformamide in a 2-L three-neck flask. The mixture was heated to 136°C and was then stirred at the same temperature for 2 hours.

The mixture was cooled to 100°C and was then filtered. The resulting filtrate was cooled to room temperature and 500 mL of water was added thereto. The mixture was stirred for 1 hour and was then filtered. The resulting solid residue was slurry-washed with water and methanol and then with hot chlorobenzene. The resulting crystals were dried under reduced pressure to give 5.84 g (yield: 78.2%) of Example Compound D16 as yellow crystals.

Mass spectrometry confirmed that Example Compound D16 had an M⁺ of 544.

The structure of Example Compound D16 was confirmed by NMR measurement.
¹H-NMR (CDCl₃) : δ(ppm) = 9.32(s, 1H), 8.86(d, 1H, J = 8.8 Hz), 8.79(s, 1H), 8.66(s, 1H), 8.34(s, 1H), 8.20(d, 1H, J = 6.8 Hz), 7.98-7.83(m, 12H), 7.78-7.62(m, 4H).

The photoluminescence emission spectrum of a solution of 1.0×10⁻⁶ mol/L of Example Compound D16 in toluene was measured with a fluorospectrophotometer, F-5400, manufactured by Hitachi, Ltd., at an excitation wavelength of 399 nm. The spectrum was a blue emission spectrum having an emission peak showing a maximum intensity at 429 nm.

### Example 2

### Synthesis of Example Compounds D50 and D51

### (1) Synthesis of intermediate compound M4

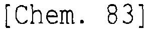

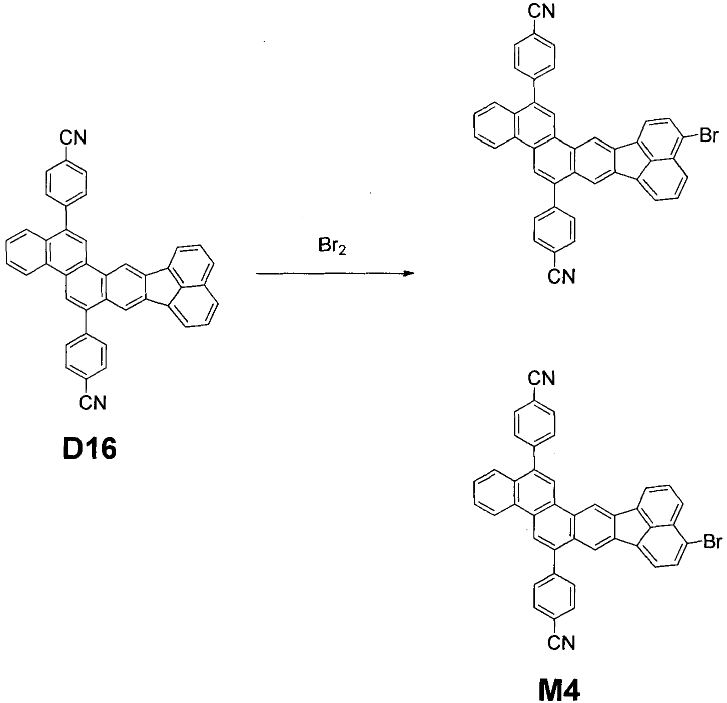

Under a nitrogen atmosphere, 5.3 g (9.52 mmol) of Example Compound D16 was added to 5300 mL of methylene chloride in a 10-L separable flask, followed by stirring for dissolution. A solution of 16.2 g (104 mmol) of bromine in methylene chloride was dropwise added to the solution, followed by stirring at room temperature for 10 hours.

A sodium thiosulfate solution was added to the resulting solution. The mixture was stirred for 1 hour and was then filtered. The resulting solid residue was slurry-washed with water and methanol and then with hot methylene chloride. The resulting crystals were dried under reduced pressure to give 4.67 g (yield: 77.2%) of intermediate compound M4 as yellow crystals.

### (2) Synthesis of Example Compounds D50 and D51

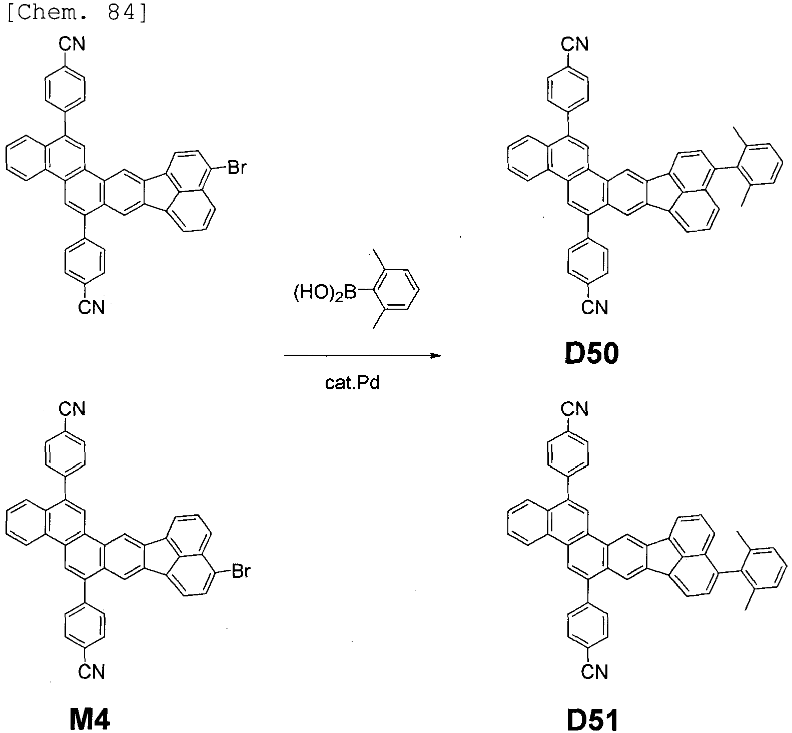

Under a nitrogen atmosphere, 1.00 g (1.58 mmol) of intermediate compound M4, 0.28 g (1.89 mmol) of 2,6-dimethylphenylboranic acid, 4.02 g (18.9 mmol) of potassium phosphate, 500 mL od N,N-dimethylformamide, and 0.18 g (0.16 mmol) of tetrakis(triphenylphosphine)palladium were sequentially placed in a 1-L three-neck flask and were heated to 100°C and then stirred at the same temperature for 11 hours.

The mixture was cooled to 80°C and was then filtered. The resulting solid residue was slurry-washed with water and methanol and then with hot chlorobenzene to give crude crystals of Example Compounds D50 and D51.

The resulting crude crystals were dissolved in 60 mL of chlorobenzene. The solution was purified by column chromatography (chlorobenzene), followed by slurry-washing with hot toluene. The resulting crystals were dried under reduced pressure to give 0.10 g (yield: 33.3%) of Example Compounds D50 and D51 as yellow crystals.

Mass spectrometry confirmed that the mixture of Example Compounds D50 and D51 had an M⁺ of 658. NMR measurement confirmed that the mixture ratio of Example Compounds D50 and D51 was 1:1.
¹H-NMR (CDCl₃) : δ(ppm) = 9.34(s, 2H), 8.87(d, 2H, J = 8.8 Hz), 8.80(s, 2H), 8.67(s, 1H), 8.66(s, 1H), 8.35(s, 1H), 8.33(s, 1H), 8.19(d, 1H, J = 6.8 Hz), 8.12(d, 1H, J = 6.8 Hz), 8.04(d, 1H, J = 6.8 Hz), 7.97-7.84(m, 20H), 7.77(t, 2H, J = 6.8 Hz), 7.66-7.38(m, 8H), 7.29(t, 2H, J = 6.8 Hz), 7.20(d, 3H, J = 6.8 Hz), 1.99(s, 6H), 1.98(s, 6H).

The photoluminescence emission spectrum of a solution of 1.0×10⁻⁶ mol/L of the mixture of Example Compounds D50 and D51 in toluene was measured with a fluorospectrophotometer, F-5400, manufactured by Hitachi, Ltd., at an excitation wavelength of 403 nm. The spectrum was a blue emission spectrum having an emission peak showing a maximum intensity at 433 nm.

### Example 3

### Synthesis of Example Compounds D62 and D63

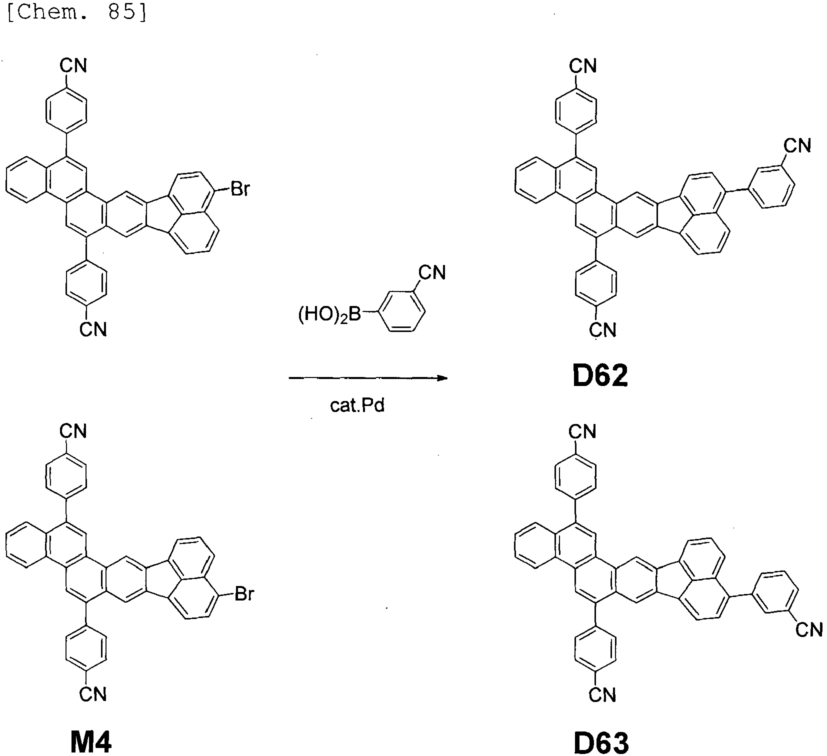

Under a nitrogen atmosphere, 0.12 g (0.19 mmol) of intermediate compound M4, 0.33 g (2.25 mmol) of 3-cyanophenylboranic acid, 0.12 g (0.57 mmol) of potassium phosphate, 30 mL of N,N-dimethylformamide, and 0.01 g (0.01 mmol) of tetrakis(triphenylphosphine)palladium were sequentially placed in a 100-mL three-neck flask and were heated to 100°C and then stirred at the same temperature for 6 hours.

The mixture was cooled to 80°C and was then filtered. The resulting solid residue was slurry-washed with water and methanol and then with hot chlorobenzene to give crude crystals of Example Compounds D62 and D63.

The resulting crude crystals were dissolved in 80 mL of chlorobenzene. The solution was purified by column chromatography (chlorobenzene), followed by slurry-washing with hot toluene. The resulting crystals were dried under reduced pressure to give 0.02 g (yield: 16.1%) of Example Compounds D62 and D63 as yellow crystals.

Mass spectrometry confirmed that the mixture of Example Compounds D62 and D63 had an M⁺ of 655. NMR measurement confirmed that the mixture ratio of Example Compounds D62 and D63 was 1:1.
¹H-NMR (CDCl₃) : δ(ppm) = 9.36(s, 1H), 9.35(s, 1H), 8.87(d, 2H, J = 8.0 Hz), 8.80(s, 2H), 8.68(s, 2H), 8.37(s, 1H), 8.36(s, 1H), 8.27(d, 1H, J = 7.2 Hz), 8.25(d, 1H, J = 7.2 Hz), 8.04-7.84(m, 26H), 7.80-7.61(m, 12H).

The photoluminescence emission spectrum of a solution of 1.0×10⁻⁶ mol/L of the mixture of Example Compounds D62 and D63 in toluene was measured with a fluorospectrophotometer, F-5400, manufactured by Hitachi, Ltd., at an excitation wavelength of 407 nm. The spectrum was a blue emission spectrum having an emission peak showing a maximum intensity at 441 nm.

### Example 4

### Synthesis of Example Compounds D64 and D65

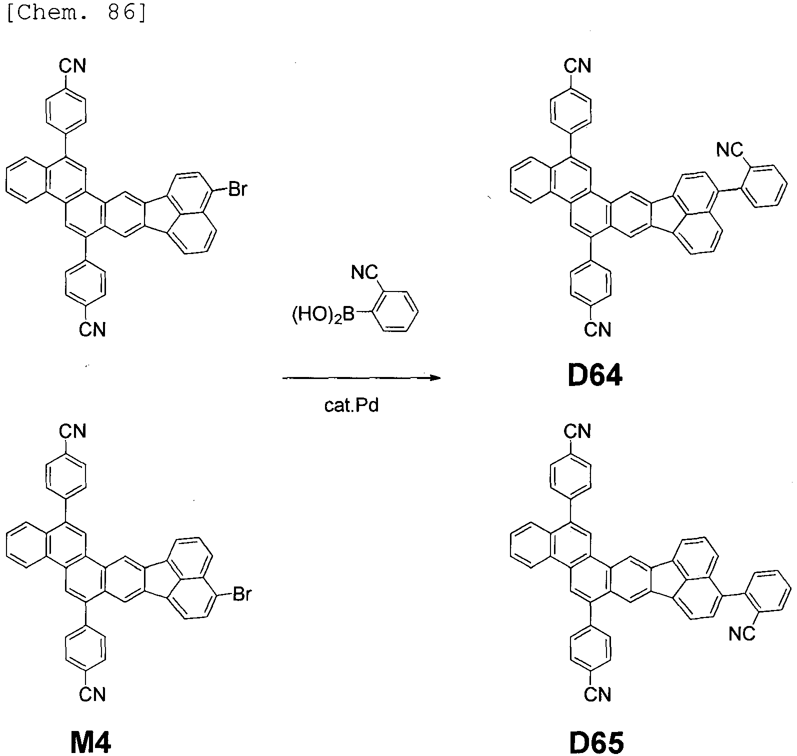

Under a nitrogen atmosphere, 1.00 g (1.58 mmol) of intermediate compound M4, 0.28 g (1.89 mmol) of 2-cyanophenylboranic acid, 2.01 g (9.47 mmol) of potassium phosphate, 500 mL of N,N-dimethylformamide, and 0.18 g (0.16 mmol) of tetrakis(triphenylphosphine)palladium were sequentially placed in a 1-L three-neck flask and were heated to 100°C and then stirred at the same temperature for 1 hour.

The mixture was cooled to 80°C and was then filtered. The resulting solid residue was slurry-washed with water and methanol to give crude crystals of Example Compounds D64 and D65.

The resulting crude crystals were dissolved in 630 mL of chlorobenzene. The solution was purified by column chromatography (chlorobenzene), followed by slurry-washing with hot toluene. The resulting crystals were dried under reduced pressure to give 0.36 g (yield: 34.4%) of Example Compounds D64 and D65 as yellow crystals.

Mass spectrometry confirmed that the mixture of Example Compounds D64 and D65 had an M⁺ of 655. NMR measurement confirmed that the mixture ratio of Example Compounds D64 and D65 was 1:1.
¹H-NMR (CDCl₃) : δ(ppm) = 9.32(s, 2H), 8.70(d, 2H, J = 8.4 Hz), 8.78(s, 2H), 8.67(s, 2H), 8.35(s, 1H), 8.33(s, 1H), 8.25(d, 1H, J = 6.8 Hz), 8.20(d, 1H, J = 6.8 Hz), 8.04(d, 2H, J = 6.8 Hz), 7.98-7.83(m, 20H), 7.80-7.57(m, 16H).

The photoluminescence emission spectrum of a solution of 1.0×10⁻⁶ mol/L of the mixture of Example Compounds D64 and D65 in toluene was measured with a fluorospectrophotometer, F-5400, manufactured by Hitachi, Ltd., at an excitation wavelength of 406 nm. The spectrum was a blue emission spectrum having an emission peak showing a maximum intensity at 441 nm.

### Example 5

### Synthesis of Example Compounds D80 and D81

### (1) Synthesis of intermediate compound M5

As shown in synthetic route 4 below, 30 g of intermediate compound M5 was synthesized by a method described in a non-patent document: Chemistry of Materials, 2009, 21(12), 2452.

### Synthetic route 4

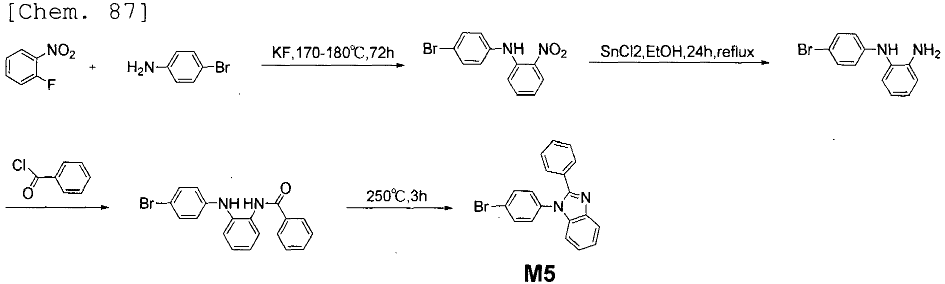

### (2) Synthesis of intermediate compound M6

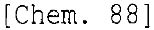

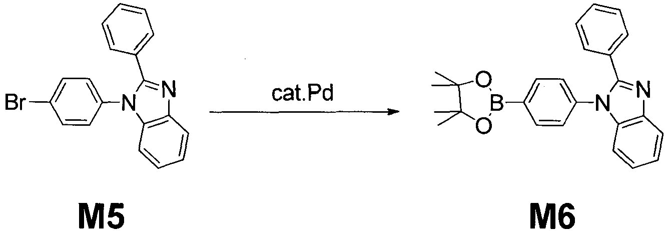

Under a nitrogen atmosphere, 5.0 g (14.3 mmol) of intermediate compound M5, 4.0 g (15.8 mmol) of bis-dioxaborolane, 4.2 g (42.8 mmol) of potassium acetate, and 0.58 g (0.71 mmol) of [1,1'-bis(diphenylphosphino)ferrocene]palladium(II) dichloride dichloromethane adduct were added to 100 mL of N,N-dimethylformamide in a 300-mL three-neck flask and were heated to 80°C and then stirred at the same temperature for 4 hours.

The mixture was cooled to room temperature and 100 mL of water added thereto. The mixture was stirred for 1 hour and was then filtered. The resulting solid residue was slurry-washed with water to give crude crystals of intermediate compound M6.

The resulting crude crystals were dissolved in toluene. The solution was purified by column chromatography (toluene/ethyl acetate = 4/1), followed by recrystallization from a solvent mixture of toluene/heptane = 4:1. The resulting crystals were dried under reduced pressure to give 4.12 g (yield: 72.7%) of intermediate compound M6.

### (3) Synthesis of Example Compounds D80 and D81

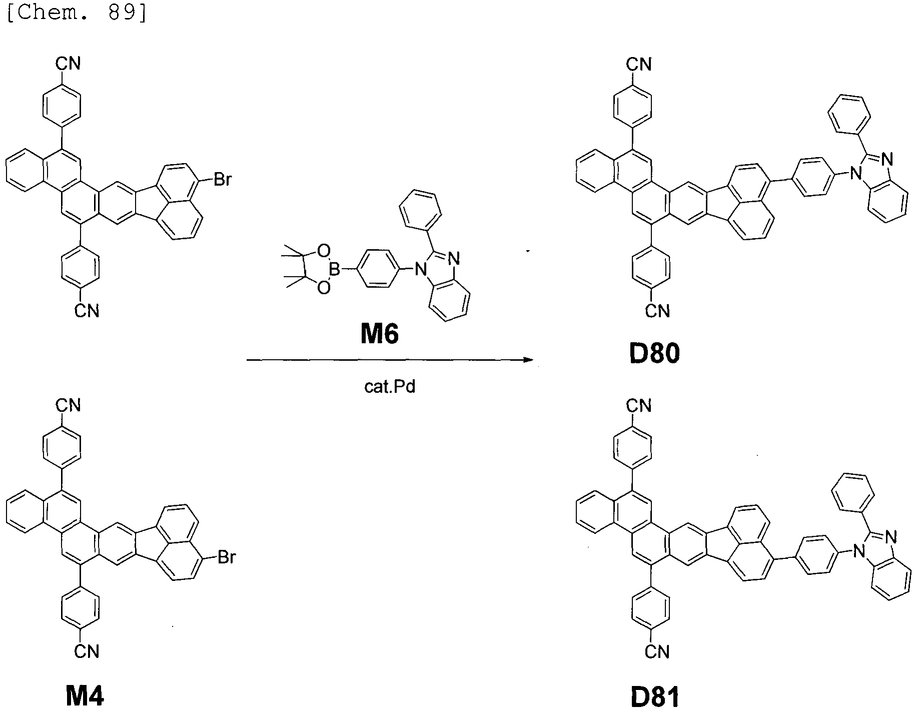

Under a nitrogen atmosphere, 0.11 g (0.17 mmol) of intermediate compound M4, 0.08 g (0.21 mmol) of intermediate compound M6, 0.11 g (0.52 mmol) of potassium phosphate, 11 \ mL of N,N-dimethylformamide, and 0.01 g (0.01 mmol) of tetrakis(triphenylphosphine)palladium were sequentially placed in a 1-L three-neck flask and were heated to 100°C and then stirred at the same temperature for 4 hours.

The mixture was cooled to room temperature and chloroform was added thereto, followed by washing with water three times. The organic layer was dried with magnesium sulfate and was then filtered. The resulting filtrate was concentrated to give crude crystals of Example Compounds D80 and D81.

The resulting crude crystals were purified by column chromatography (toluene/ethyl acetate = 10/1), followed by slurry-washing with hot methanol. The resulting crystals were dried under reduced pressure to give 0.091 g (yield: 63.6%) of Example Compounds D80 and D81 as yellow crystals.

Mass spectrometry confirmed that the mixture of Example Compounds D80 and D81 had an M⁺ of 822. NMR measurement confirmed that the mixture ratio of Example Compounds D80 and D81 was 1:1.
¹H-NMR (CDCl₃) : δ(ppm) = 9.38(s, 1H), 9.37(s, 1H), 8.88(d, 2H, J = 8.8 Hz), 8.82(s, 1H), 8.81(s, 1H), 8.69(s, 2H), 8.38(s, 1H), 8.37(s, 1H), 8.29(d, 1H, J = 6.8 Hz), 8.26(d, 1H, J = 6.8 Hz), 8.07-7.64(m, 38H), 7.51(d, 2H, J = 8.8 Hz), 8.50(d, 2H, J = 8.8 Hz), 7.44-7.32(m, 14H).

The photoluminescence emission spectrum of a solution of 1.0×10⁻⁶ mol/L of the mixture of Example Compounds D80 and D81 in toluene was measured with a fluorospectrophotometer, F-5400, manufactured by Hitachi, Ltd., at an excitation wavelength of 408 nm. The spectrum was a blue emission spectrum having an emission peak showing a maximum intensity at 443 nm.

### Example 6

### Synthesis of Example Compounds D36 and D37

Reaction and purification were performed as in Example 2 except that intermediate compound M7 was used in place of 2,6-dimethylphenylboranic acid used in Example 2.

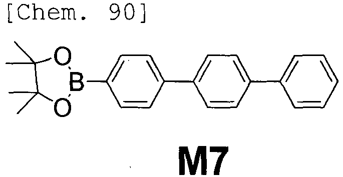

The photoluminescence emission spectrum of a solution of 1.0×10⁻⁶ mol/L of the mixture of Example Compounds D36 and D37 in toluene was measured with a fluorospectrophotometer, F-5400, manufactured by Hitachi, Ltd., at an excitation wavelength of 410 nm. The spectrum was a blue emission spectrum having an emission peak showing a maximum intensity at 445 nm.

### Comparative Example 1

Compounds R1 and R2 were synthesized as comparative examples, and photoluminescence and absorption spectra thereof were measured and were used for comparison of quantum efficiency. The absorption spectra were measured with a spectrophotometer, UV-570, manufactured by JASCO Corp. The quantum efficiency of Example Compound D16 was defined as 1.0, and those of other compounds were expressed as relative intensities thereof. The results are shown in Table 6.

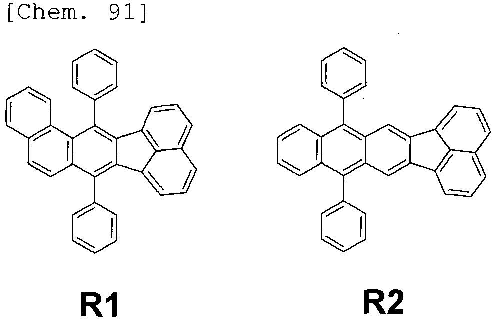

**[Table 6]**

| | Emission wavelength (nm) | Quantum yield |
|---|---|---|
| Example compound D16 | 429 | 1.00 |
| Example compound D50,51 | 433 | 1.02 |
| Example compound D62,63 | 441 | 1.05 |
| Example compound D64,65 | 441 | 1.04 |
| Example compound D80,81 | 443 | 1.06 |
| Comparative Example compound R1 | 430 | 0.98 |
| Comparative Example compound R2 | 459 | 1.06 |

All of the quantum efficiencies of five example compounds of the present invention were higher than that of Comparative Compound R1.

As shown in Fig. 2, the photoluminescence spectrum of Comparative Compound R1 was compared to that of Example Compound D16 of the present invention. The height ratio of the second peak to the first peak in Comparative Compound R1 was 0.9, but the ratio in Example Compound D16 of the present invention was low, 0.7, and the height ratios of subsequent peaks were similarly low.

That is, in Example Compound D16 of the present invention, the peak in the blue light wavelength region is the highest, and the peaks in regions other than the blue light wavelength region are low.

The results demonstrate that Example Compound D16 of the present invention has a higher emission intensity in the blue light wavelength region compared to Comparative Compound R1.

Herein, the term "first peak" refers to a peak having the highest intensity in an emission spectrum. The peaks in descending order according to intensity are referred to as the second, third, and so forth peaks.

The light emission peak wavelength of Comparative Compound R2 was compared to that of Example Compound D16 of the present invention. The peak wavelength of Comparative Compound R2 was 459 nm, but the peak wavelength of Example Compound D16 was 429 nm. That is, the light emission peak wavelength of Example Compound D16 of the present invention is shorter than that of Comparative Compound R2.

The basic skeleton itself of the fused polycyclic compound of the present invention emits shorter wavelength light compared Comparative Compound R2. Consequently, the freedom in selection of substituents is high even in the case of maintaining the blue light emission after introduction of the substituents.

Specific examples of such a compound include four Example Compounds shown in Table 6 prepared by introducing aryl and heterocyclic group substituents into Example Compound D16 of the present invention. These compounds all emit light with a maximum emission wavelength of 450 nm or less.

That is, in the fused polycyclic compound of the present invention, the basic skeleton itself emits light in a blue region. Emission of blue light of 450 nm or less and high quantum efficiency can be achieved by appropriately selecting the introduction position of a substituent and the type of the substituent.

### Examples 7 to 13

In these examples, organic light-emitting devices each having an anode, a hole-transporting layer, a light-emitting layer, a hole/exciton-blocking layer, an electron-transporting layer, and a cathode disposed in this order on a substrate were produced by the following method.

A film of ITO having a thickness of 100 nm was formed on a glass substrate by sputtering as an anode. The glass substrate provided with the anode was used as a transparent electrically conductive support substrate (ITO substrate).

On this ITO substrate, organic compound layers and electrode layers shown below were successively formed by resistance heating vacuum vapor deposition in a vacuum chamber of 10⁻⁵ Pa. On this occasion, the area of electrodes facing each other was adjusted to be 3 mm². When two guest materials are used, the guest is a mixture of isomers having substituents at different positions at a ratio of 1:1. The layers were:
hole-injecting layer (70 nm): compound G1,
hole-transporting layer (45 nm): compound G2,
light-emitting layer (20 nm): host material: Compound G3,
guest material: Example Compound shown in Table 7 (weight ratio: 1%),
hole/exciton-blocking layer (5 nm): compound G4,
electron-transporting layer (20 nm): compound G5,
metal electrode layer 1 (1 nm): LiF, and
metal electrode layer 2 (100 nm): Al.

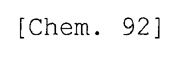

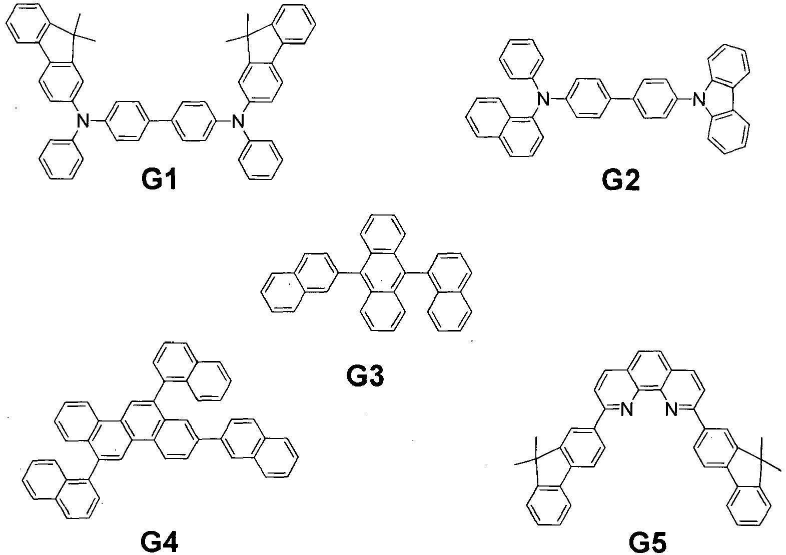

As the properties of the EL device, the current-voltage properties were measured with a micro-ammeter, 4140B, manufactured by Hewlett-Packard Company, and the light emission luminance was measured with a color luminance meter, BM7, manufactured by Topcom Corp.

The properties of EL devices of Examples 7 to 13 are shown in Table 7.

### Examples 14 and 15

In these examples, organic light-emitting devices were produced as in the method shown in Examples 7 to 13 except that compound G6 was used in the hole/exciton-blocking layer.

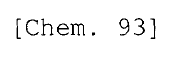

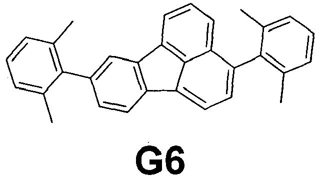

### Examples 16 and 17

In these examples, organic light-emitting devices were produced as in the method shown in Examples 7 to 13 except that compound G7 was used in the hole/exciton-blocking layer.

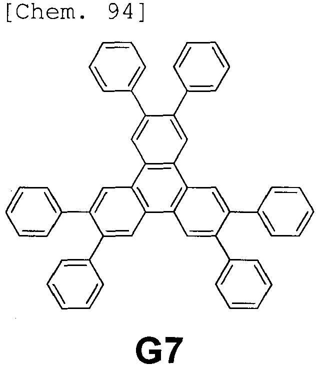

### Comparative Examples 2 and 3

In these comparative examples, organic light-emitting devices were produced as in the method shown in Examples 7 to 13 except that Comparative Compounds R1 and R2 were used as the guest materials of the light-emitting layers.

As the properties of the EL device, the current-voltage properties were measured with a micro-ammeter, 4140B, manufactured by Hewlett-Packard Company, and the light emission luminance was measured with a color luminance meter, BM7, manufactured by Topcom Corp.

The properties of EL devices of Comparative Examples 2 and 3 are shown in Table 7.

**[Table 7]**

| | Guest | Hole/exciton blocking layer | External quantum efficiency @10mA/cm2 | CIE chromaticity (X, Y) |
|---|---|---|---|---|
| Example 7 | D16 | G4 | 3.6 | (0.16,0.06) |
| Example 8 | D24 | G4 | 3.5 | (0.16,0.08) |
| Example 9 | D27 | G4 | 3.8 | (0.15,0.12) |
| Example 10 | D36, D37 | G4 | 5.2 | (0.15,0.13) |
| Example 11 | D50,D51 | G4 | 4.6 | (0.15,0.07) |
| Example 12 | D64,D65 | G4 | 4.9 | (0.15,0.11) |
| Example 13 | D70,D71 | G4 | 5.0 | (0.15,0.12) |
| Example 14 | D16 | G6 | 6.1 | (0.15,0.07) |
| Example 15 | D80,D81 | G6 | 6.2 | (0.15,0.10) |
| Example 16 | D62,D63 | G7 | 6.8 | (0.15,0.11) |
| Example 17 | D80,D81 | G7 | 6.7 | (0.15,0.11) |
| Comparative Example 2 | R1 | G4 | 2.6 | (0.16,0.06) |
| Comparative Example 3 | R2 | G4 | 4.0 | (0.15,0.20) |

### Results and conclusion

The organic compound of the present invention is a novel compound showing a high quantum efficiency and emitting blue light, and the compound can provide an organic light-emitting device emitting light with a high color purity and high luminance.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

This application claims the benefit of Japanese Patent Application No. 2012-174880, filed August 7, 2012, which is hereby incorporated by reference herein in its entirety.

### Reference Signs List

- 8: TFT device
- 11: anode
- 12: organic compound layer
- 13: cathode

## Claims

1. A fused polycyclic compound represented by Formula [1]: 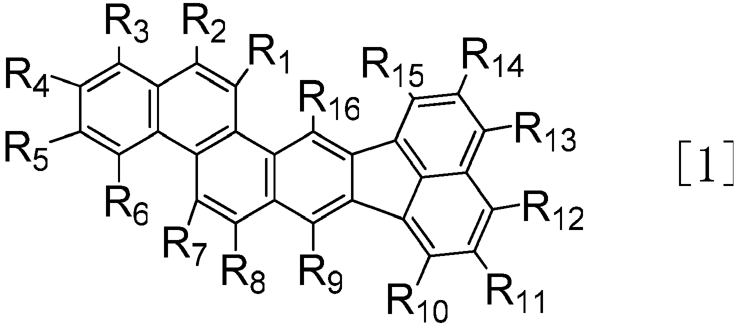 wherein, R₁ to R₁₆ each independently represent a group selected from a hydrogen atom, a cyano group, substituted or unsubstituted alkyl groups, substituted or unsubstituted aryl groups, and substituted or unsubstituted heterocyclic groups.

2. The fused polycyclic compound according to Claim 1, wherein
at least two of R₂, R₄, R₈, R₁₂, and R₁₃ are electron-withdrawing substituents.

3. An organic light-emitting device comprising:
a pair of electrodes; and
an organic compound layer disposed between the pair of electrodes, wherein
the organic compound layer includes a fused polycyclic compound according to Claim 1 or 2.

4. The organic light-emitting device according to Claim 3, wherein
the organic compound layer is a light-emitting layer including a host material and a guest material; and
the guest material is the fused polycyclic compound.

5. The organic light-emitting device according to Claim 3 or 4, wherein
the organic compound layer is a light-emitting layer including a plurality of guest materials;
at least one of the plurality of guest materials is the fused polycyclic compound; and the device emits white light.

6. A display apparatus comprising:
a plurality of pixels, wherein
at least one of the plurality of pixels includes an organic light-emitting device according to any one of Claims 3 to 5 and an active device connected to the organic light-emitting device.

7. An image information processing apparatus comprising:
an image input section for inputting image information; and
a display section for displaying an image, wherein
the display section is a display apparatus according to Claim 6.

8. A lighting system comprising:
an organic light-emitting device according to any one of Claims 3 to 5; and
an AC/DC converter circuit connected to the organic light-emitting device.

9. An image forming apparatus comprising:
a photosensitive member;
a charging unit for charging a surface of the photosensitive member;
an exposure unit for forming an electrostatic latent image by exposing the photosensitive member to light; and
a developing unit for developing the electrostatic latent image formed on the surface of the photosensitive member, wherein
the exposure unit includes an organic light-emitting device according to any one of Claims 3 to 5.

10. An exposure unit for exposing a photosensitive member to light, the unit comprising:
organic light-emitting devices according to any one of Claims 3 to 5, wherein
the organic light-emitting devices are arranged in a line.

## Patentansprüche

1. Kondensierte polyzyklische Verbindung, die durch Formel [1] dargestellt ist: 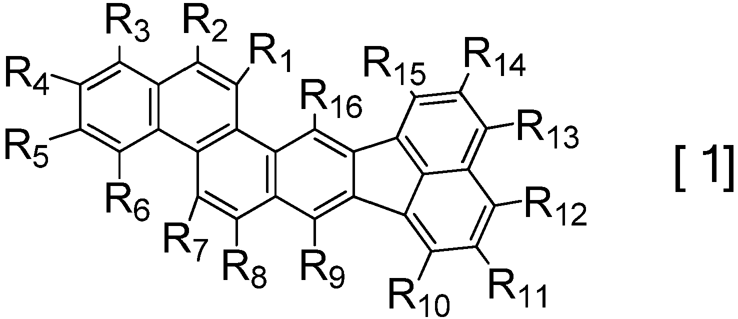 wobei R₁ bis R₁₆ jeweils unabhängig eine Gruppe ausgewählt aus einem Wasserstoffatom, einer Cyanogruppe, substituierten oder unsubstituierten Alkylgruppen, substituierten oder unsubstituierten Arylgruppen und substituierten oder unsubstituierten heterozyklischen Gruppen darstellen.

2. Kondensierte polyzyklische Verbindung nach Anspruch 1, wobei zumindest zwei der R₂, R₄, R₈, R₁₂ und R₁₃ elektronenentziehende Substituenten sind.

3. Organische licht-emittierende Vorrichtung umfassend:
ein Elektrodenpaar; und
eine zwischen dem Elektrodenpaar eingefügte organische Verbindungsschicht, wobei
die organische Verbindungsschicht eine kondensierte polyzyklische Verbindung nach Anspruch 1 oder 2 enthält.

4. Organische licht-emittierende Vorrichtung nach Anspruch 3, wobei
die organische Verbindungsschicht eine licht-emittierende Schicht ist,
die ein Wirtsmaterial und ein Gastmaterial enthält; und
das Gastmaterial die kondensierte polyzyklische Verbindung ist.

5. Organische licht-emittierende Vorrichtung nach Anspruch 3 oder 4, wobei
die organische Verbindungsschicht eine licht-emittierende Schicht ist,
die eine Vielzahl von Gastmaterialen enthält;
zumindest eines der Vielzahl von Gastmaterialien die kondensierte polyzyklische Verbindung ist; und
die Vorrichtung weißes Licht emittiert.

6. Anzeigegerät umfassend:
eine Vielzahl von Pixeln, wobei
zumindest eines der Vielzahl von Pixeln eine organische licht-emittierende Vorrichtung nach einem der Ansprüche 3 bis 5 und eine aktive Vorrichtung, die mit der organischen licht-emittierenden Vorrichtung verbunden ist, enthält.

7. Bildinformationsverarbeitungsgerät umfassend:
eine Bildeingabesektion zum Eingeben von Bildinformation; und
eine Anzeigesektion zum Anzeigen eines Bildes, wobei die Anzeigesektion ein Anzeigegerät nach Anspruch 6 ist.

8. Beleuchtungssystem umfassend:
eine organische licht-emittierende Vorrichtung nach einem der Ansprüche 3 bis 5; und eine AC/DC Konverterschaltung, die mit der organischen licht-emittierenden Vorrichtung verbunden ist.

9. Bilderzeugungsgerät umfassend:
ein photosensitives Element;
eine Ladeeinheit zum Laden einer Oberfläche des photosensitiven Elements;
eine Belichtungseinheit zum Erzeugen eines elektrostatisch latenten Bildes durch Belichten des photosensitiven Elements; und
eine Entwicklereinheit zum Entwickeln des auf der Oberfläche des photosensitiven Elements erzeugten elektrostatischen latenten Bildes, wobei die Belichtungseinheit eine organische licht-emittierende Vorrichtung nach einem der Ansprüche 3 bis 5 enthält.

10. Belichtungseinheit zum Belichten eines photosensitiven Elements, die Einheit umfassend:
organisch licht-emittierende Vorrichtung nach einem der Ansprüche 3 bis 5, wobei
die organischen licht-emittierenden Vorrichtungen in einer Linie angeordnet sind.

## Revendications

1. Composé polycyclique condensé représenté par la formule [1] : 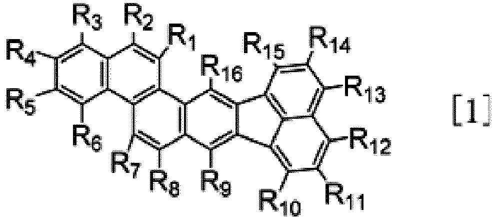 dans laquelle, R₁ à R₁₆ représentent chacun indépendamment un groupe choisi parmi un atome d'hydrogène, un groupe cyano, des groupes alkyle substitués ou non substitués, des groupes aryle substitués ou non substitués et des groupes hétérocycliques substitués ou non substitués.

2. Composé polycyclique condensé selon la revendication 1, dans lequel au moins deux de R₂, R₄, R₈, R₁₂ et R₁₃ sont des substituants attracteurs d'électrons.

3. Dispositif luminescent organique comprenant :
une paire d'électrodes ; et
une couche de composé organique disposée entre la paire d'électrodes, où
la couche de composé organique comprend un composé polycyclique condensé selon la revendication 1 ou 2.

4. Dispositif luminescent organique selon la revendication 3, dans lequel
la couche de composé organique est une couche luminescente comprenant un matériau hôte et un matériau invité ; et
le matériau invité est le composé polycyclique condensé.

5. Dispositif luminescent organique selon la revendication 3 ou 4, dans lequel
la couche de composé organique est une couche luminescente comprenant une pluralité de matériaux invités ;
au moins un de la pluralité de matériaux invités est le composé polycyclique condensé ; et
le dispositif émet de la lumière blanche.

6. Appareil d'affichage comprenant :
une pluralité de pixels, où
au moins un de la pluralité de pixels comprend un dispositif luminescent organique selon l'une quelconque des revendications 3 à 5 et un dispositif actif connecté au dispositif luminescent organique.

7. Appareil de traitement d'informations d'images comprenant :
une section d'entrée d'informations d'images pour entrer les informations d'images ; et
une section d'affichage pour afficher une image, où
la section d'affichage est un appareil d'affichage selon la revendication 6.

8. Système d'éclairage comprenant :
un dispositif luminescent organique selon l'une quelconque des revendications 3 à 5 ; et
un circuit convertisseur AC/DC connecté au dispositif luminescent organique.

9. Appareil formant une image comprenant :
un élément photosensible ;
une unité de chargement pour charger une surface de l'élément photosensible ;
une unité d'exposition pour former une image latente électrostatique en exposant l'élément photosensible à la lumière ; et
une unité de développement pour développer l'image latente électrostatique formée sur la surface de l'élément photosensible, où
l'unité d'exposition comprend un dispositif luminescent organique selon l'une quelconque des revendications 3 à 5.

10. Unité d'exposition pour exposer un élément photosensible à la lumière, l'unité comprenant :
des dispositifs luminescents organiques selon l'une quelconque des revendications 3 à 5, où
les dispositifs luminescents organiques sont disposés en ligne.
